# EUROPEAN PATENT APPLICATION

(11) **EP 4 495 135 A1**
(43) Date of publication of application: **22.01.2025**
(21) Application number: 23784290.1
(22) Date of filing: 04.04.2023
(51) Int. Cl.: C07K 14/605, A61K 38/26, A61P 3/00, A61P 3/10

(54) **PHARMACEUTICAL USE OF POLYPEPTIDE IN PREPARATION OF DRUGS FOR TREATING AND/OR PREVENTING DIABETES AND OBESITY AND RELATED DISEASES THEREOF**

(30) Priority: 07.04.2022 CN 202210365272; 13.07.2022 CN 202210823155; 23.03.2023 CN 202310301962
(71) Applicant: Guangdong Raynovent Biotech Co., Ltd., Huangpu District Guangzhou 510700 (CN)
(72) Inventor: CHEN, Xiaoxin, Guangzhou, Guangdong 510700 (CN); LIU, Zhuowei, Guangzhou, Guangdong 510700 (CN); CHEN, Minglu, Guangzhou, Guangdong 510700 (CN); HUANG, Jianzhou, Guangzhou, Guangdong 510700 (CN); PANG, Daolin, Guangzhou, Guangdong 510700 (CN); ZHANG, Qianru, Guangzhou, Guangdong 510700 (CN); LIU, Miao, Guangzhou, Guangdong 510700 (CN); REN, Yingshan, Guangzhou, Guangdong 510700 (CN); LONG, Chaofeng, Guangzhou, Guangdong 510700 (CN)
(74) Representative: Potter Clarkson
(86) International application number: PCT/CN2023/086327
(87) International publication number: WO 2023/193727

(57) **Abstract**

The pharmaceutical use of a series of polypeptides in the preparation of a drug for treating and/or preventing diabetes, obesity, and related diseases thereof, which specifically relates to a polypeptide having a sequence as shown in formula (II).
YAibEGT FTSDY SIAibLD KIAQK AFVKW LIAGG PSSGA PPPS₀(II).

## Description

### TECHNICAL FIELD

The present application belongs to the field of biomedicine, and specifically relates to a pharmaceutical use of a polypeptide having a sequence as shown in formula (II) in the preparation of a drug for treating and/or preventing diabetes, obesity, and related diseases thereof.

### BACKGROUND

The global incidence of non-alcoholic fatty liver disease (NAFLD) is as high as 25%, with the global incidence of non-alcoholic steatohepatitis (NASH) accounting for approximately 3% to 8%. Some patients with NASH will further progress to cirrhosis and liver cancer, which is currently one of the leading causes of end-stage liver disease and liver transplantation. The pathogenesis of NASH is complex, and there is no FDA-approved drug for the treatment of this disease at present. Several preclinical studies have found that glucose-dependent insulinotropic polypeptide (GIP)/glucagon-like peptide-1 (GLP-1) dual agonists can be used for the treatment of NASH. Clinical studies of Tirzepatide, a GLP-1/GIP dual agonist under the investigation by Eli Lilly and Company, have demonstrated that it can improve NASH-related transaminase and other relevant markers, showing its potential as a treatment for NASH.

GLP-1 agonists can be used to treat NASH synergistically through multiple pathways. In vivo studies in animals have found that GLP-1 agonists can inhibit appetite in brain, delay gastric emptying, reduce hepatic gluconeogenesis, increase glucose consumption in muscle tissue, and consequently reduce body weight. Meanwhile, GLP-1 agonists can reduce the circulating levels of tumor necrosis factor (TNF)-α, interleukin IL-1β, IL-6, CD163, and hsCRP, thus achieving an anti-inflammatory effect. In addition, GLP-1 agonists can inhibit fatty acid synthase, inhibit LDL uptake, and reduce adipose tissue blood flow, thereby improving symptoms of NASH through multiple ways. GIP is a polypeptide secreted by neuroendocrine K cells in small intestine, and activation of GIPR can enhance lipid metabolism and further alleviate hepatic lipid synthesis on the basis of GLP-1 agonist treatment. Therefore, GLP-1/GIP dual agonists have a synergistic effect in the treatment of NASH.

Moreover, it has also been found in the studies that GLP-1/GIP dual agonists have excellent effects in mediating organismal glucose metabolism and lipid metabolism, among other aspects, indicating that compounds directed against this target may have the prospect of being developed into drugs for preventing and/or treating a range of specific diseases associated with abnormal glucose and/or lipid metabolism, such as diabetes, obesity and complications (related diseases) caused thereby.

### SUMMARY OF THE INVENTION

The present application provides a pharmaceutical use of a polypeptide having a sequence as shown in formula (II) in the preparation of a drug for treating and/or preventing diabetes, obesity, and related diseases thereof,
YAibEGT FTSDY SIAibLD KIAQK AFVKW LIAGG PSSGA PPPS₀ **(II)**
which has modifications as below:
1) the amino groups on the side chains of lysine residues at positions i and i+3 or the amino groups on the side chains of lysine residues at positions j and j+4 are attached to 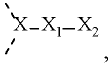 wherein i is 17 (i.e., when i is 17, isoleucine residue at position 17 is replaced by lysine residue first, and the amino group on the side chain of the replaced lysine residue is then attached to the amino group on the side chain of lysine residue at position 20, and wherein the amino groups on the side chains of lysine residues at positions 17 and 20 are attached to X respectively, and the amino groups are attached through 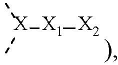 or wherein j is 20; and
2) additional 0 to 2 amino acids of the polypeptide of the sequence as shown in formula (II) are replaced;
   wherein,
   Aib has a structure of 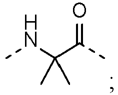
   S₀ is selected from the group consisting of serine (S, (S)-2-amino-3-hydroxypropionic acid) which has a structue of 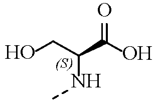 and (S)-2-amino-3-hydroxypropionamide which has a structue of 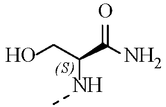 (i.e., the amino acid (serine) at position 39 is optionally amidated to a C-terminal primary amide);
   X is selected from the group consisting of 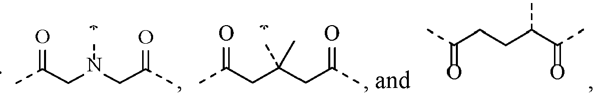
   wherein, "*" indicates the position attached to X₁;
      X₁ is selected from the group consisting of a single bond, -C(=O)-, -O-C(=O)-, and -N(R₁)-C(=O)-;
      R₁ is selected from the group consisting of H and C₁₋₃ alkyl;
      X₂ is selected from the group consisting of 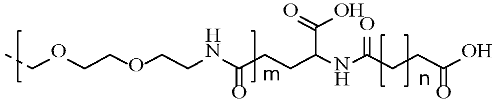 and 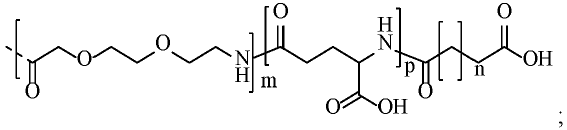
         m is selected from 2, 3, and 4;
         n is selected from 15, 16, 17, 18, and 19;
         p is selected from 1 and 2.
The polypeptide sequence as shown in formula (II) is as shown in SEQ ID NO. 1.

The present application provides a pharmaceutical use of a polypeptide having a sequence as shown in formula (P) in the preparation of a drug for treating and/or preventing diabetes, obesity, and related diseases thereof,
YAibEGT FTSDY SIAibLD KKAQK AFVKW LIAGG PSSGA PPPS₀ **(P)**
which has modifications as below:
1) the amino groups on the side chains of lysine residues at positions 17 and 20 are attached to 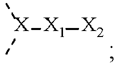 and
2) 0 to 2 amino acids of the polypeptide of the sequence as shown in formula (P) are replaced; wherein,
   Aib has a structure of 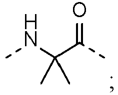
   S₀ is selected from the group consisting of serine (S, (S)-2-amino-3-hydroxypropionic acid) which has a structue of 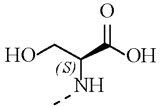 and (S)-2-amino-3-hydroxypropionamide which has a structue of 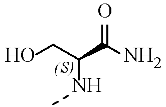 (i.e., the amino acid (serine) at position 39 is optionally amidated to a C-terminal primary amide);
   X is selected from the group consisting of 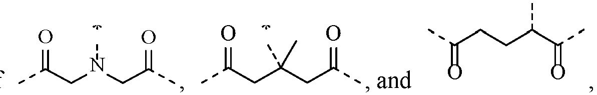
   wherein, "*" indicates the position attached to X₁;
      X₁ is selected from the group consisting of a single bond, -C(=O)-, -O-C(=O)-, and -N(R₁)-C(=O)-;
      R₁ is selected from the group consisting of H and C₁₋₃ alkyl;
      X₂ is selected from the group consisting of 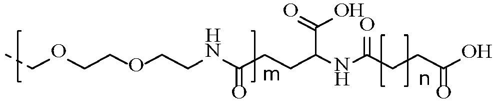 and 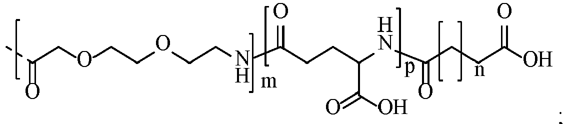
         m is selected from 2, 3, and 4;
         n is selected from 15, 16, 17, 18, and 19;
         p is selected from 1 and 2.
The polypeptide sequence as shown in formula (P) is as shown in SEQ ID NO. 2.

In some embodiments of the present application, 0 to 2 amino acids at positions 21, 23, and 24 of the polypeptide are replaced, with other variables being defined herein.

In some embodiments of the present application, the polypeptide has a sequence selected from the group consisting of sequences as shown in formulas (II-1), (II-2), (II-3), (II-4), (III-6), (IV-7), (IV-8), (IV-9), and (IV-10). The polypeptide of formulas (II-1), (II-2), (II-3), (II-4), (III-6), (IV-7), (IV-8), (IV-9), and (IV-10) are as shown in SEQ ID NOs. 3-11, respectively.
YAibEGT FTSDY SIAibLD KIAQK AFVKW LIAGG PSSGA PPPS-NH₂ **(II-1)**
YAibEGT FTSDY SIAibLD KIAQK EFVKW LIAGG PSSGA PPPS-NH₂ **(II-2)**
YAibEGT FTSDY SIAibLD KIAQK AFIKW LIAGG PSSGA PPPS-NH₂ **(II-3)**
YAibEGT FTSDY SIAibLD KIAQK AFVKW LLAGG PSSGA PPPS-NH₂ **(II-4)**
YAibEGT FTSDY SIAibLD KKAQK AFVEW LIAGG PSSGA PPPS-NH₂ **(III-6)**
YAibEGT FTSDY SIAibLD KKAQK AFVQW LIAGG PSSGA PPPS-NH₂ **(IV-7)**
YAibEGT FTSDY SIAibLD KKAQK AFVAW LIAGG PSSGA PPPS-NH₂ **(IV-8)**
YAibEGT FTSDY SIAibLD KKAQK AFVIW LIAGG PSSGA PPPS-NH₂ **(IV-9)**
YAibEGT FTSDY SIAibLD KKAQK EFVEW LIAGG PSSGA PPPS-NH₂ **(IV-10)**
which have modifications as below:
the amino groups on the side chains of lysine residues at positions 17 and 20 or the amino groups on
the side chains of lysine residues at positions 20 and 24 are attached to 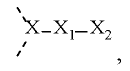
wherein,
   the serine at position 39 is amidated to a C-terminal primary amide; Aib, X, X₁, and X₂ are as defined herein.

In some embodiments of the present application, the above R₁ is selected from H, with other variables being defined herein.

In some embodiments of the present application, the above X₁ is selected from a single bond, - C(=O)-, -O-C(=O)-, and -NH-C(=O)-, with other variables being defined herein.

In some embodiments of the present application, the above m is selected from 2, with other variables being defined herein.

In some embodiments of the present application, the above n is selected from 15 and 17, with other variables being defined herein.

In some embodiments of the present application, the above X₂ is selected from the group consisting of 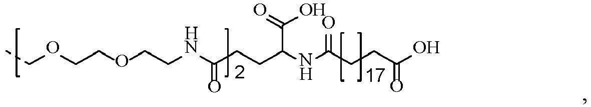 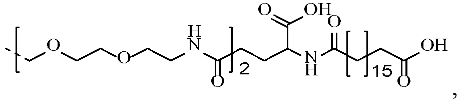 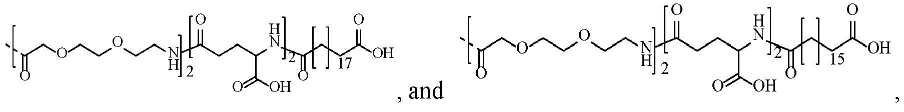 with other variables being defined herein.

In some embodiments of the present application, the above X₂ is selected from the group consisting of 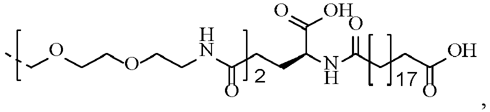 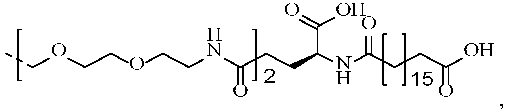 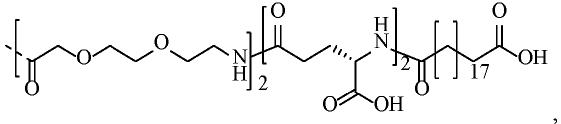 and 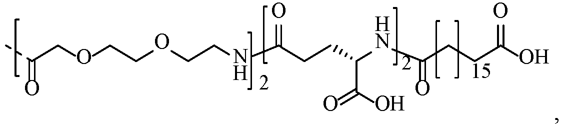 with other variables being defined herein.

In some embodiments of the present application, the amino groups on the side chains of lysine residues at positions i and i+3 or the amino groups on the side chains of lysine residues at positions j and j+4 are attached to 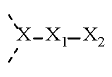 to form 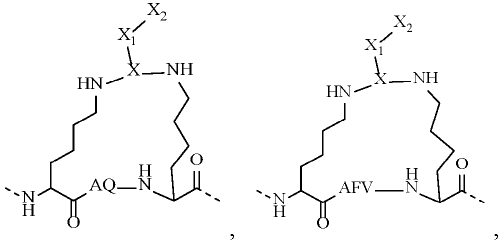 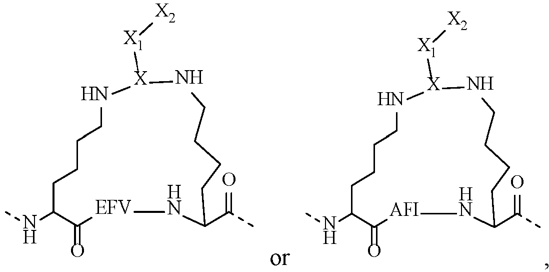 with other variables being defined herein.

In some embodiments of the present application, the amino groups on the side chains of lysine residues at positions i and i+3 or the amino groups on the side chains of lysine residues at positions j and j+4 are attached to 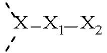 to form 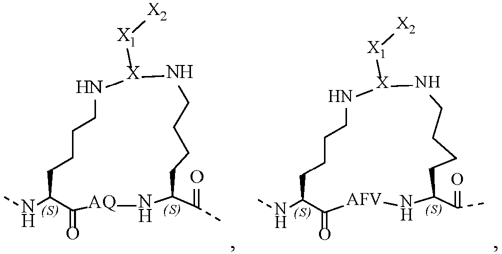 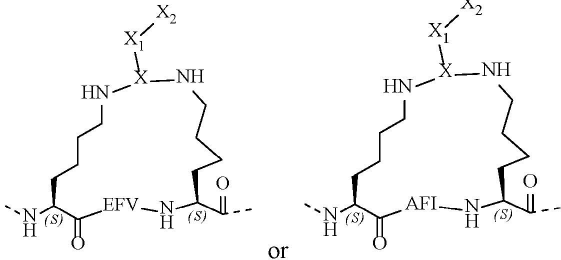 , with other variables being defined herein.

In some embodiments of the present application, the above 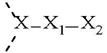 is selected from the group consisting of 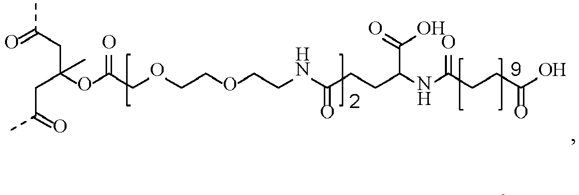 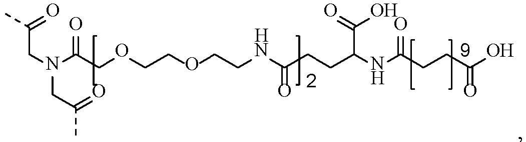 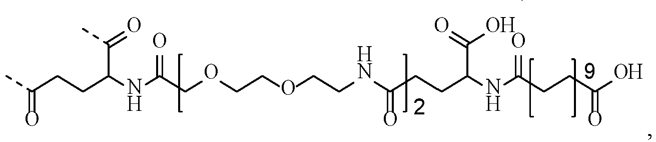 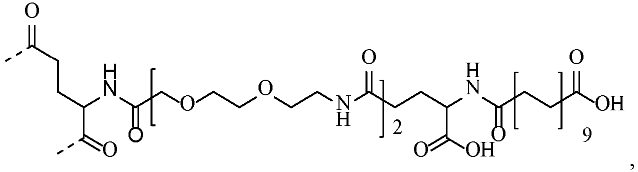 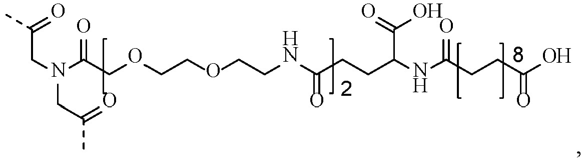 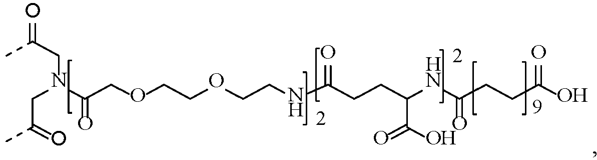 and 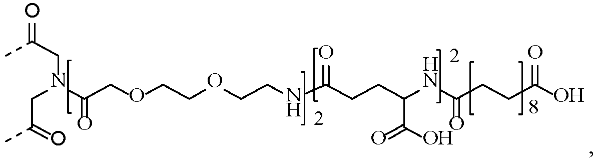 with other variables being defined herein.

In some embodiments of the present application, the above group consisting of 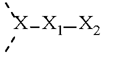 is selected from the 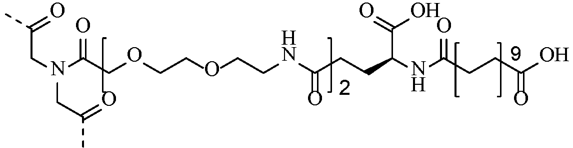 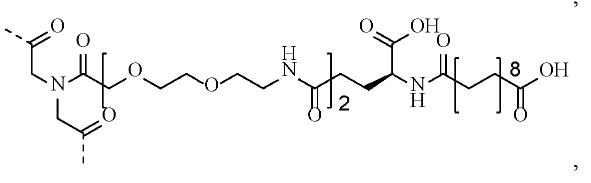 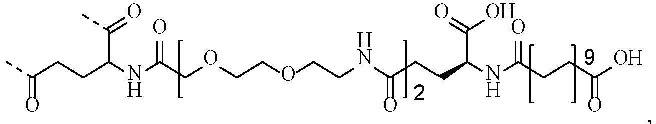 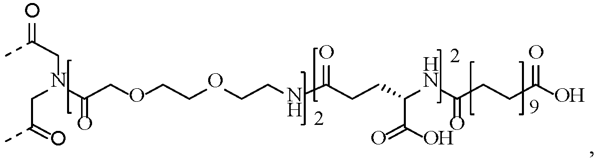 and 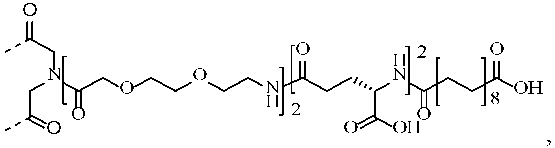 with other variables being defined herein.

The present application also encompasses some embodiments formed by any combination of the above variables.

The present application further provides a pharmaceutical use of a polypeptide having a sequence selected from the group consisting of sequences as shown in the formulas below in the preparation of a drug for treating and/or preventing diabetes, obesity, and related diseases thereof, 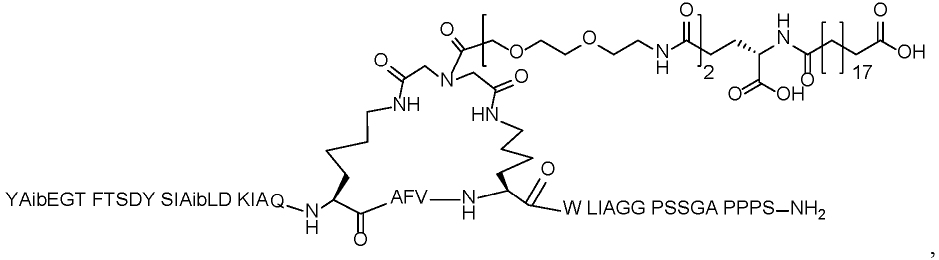 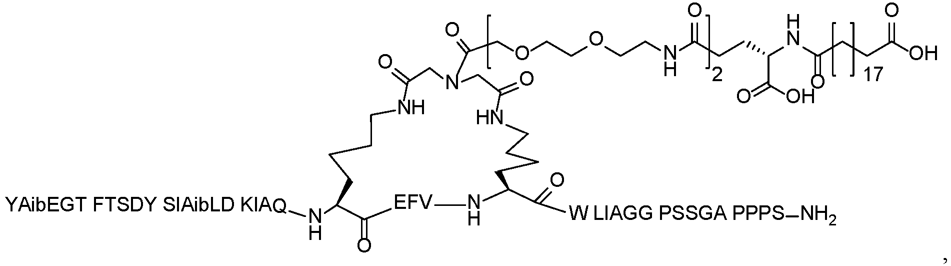 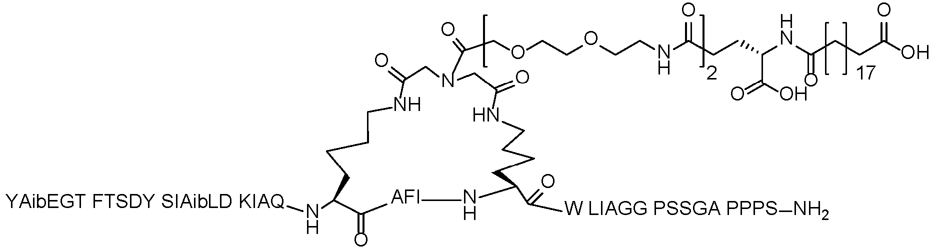 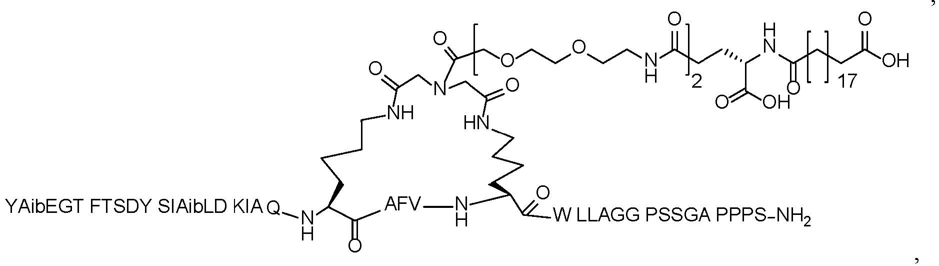 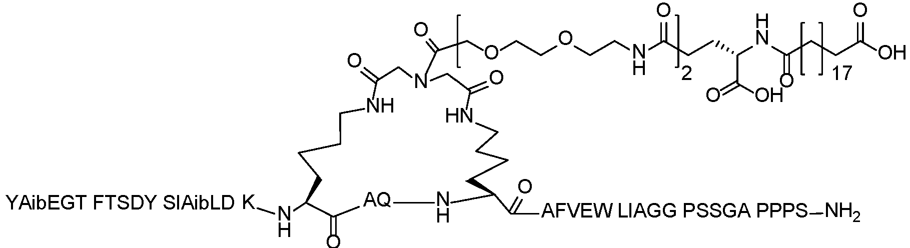 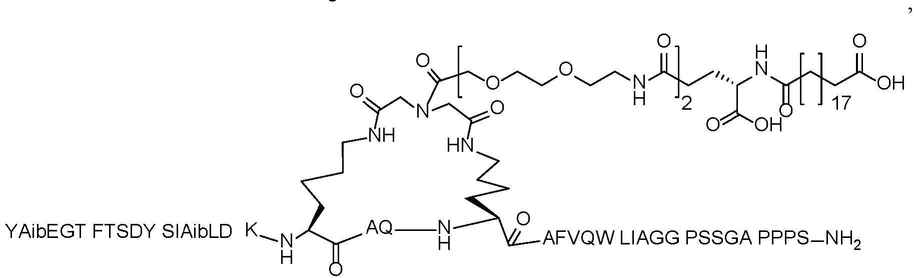 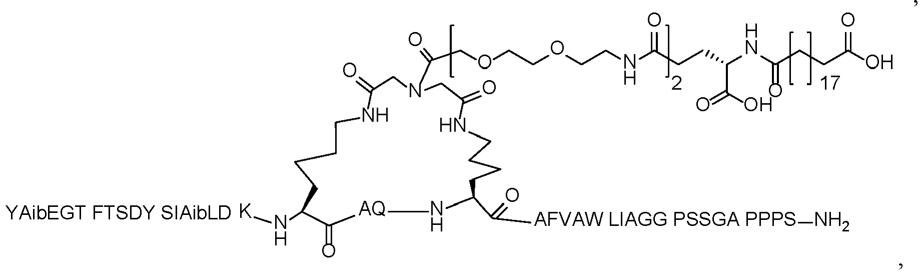 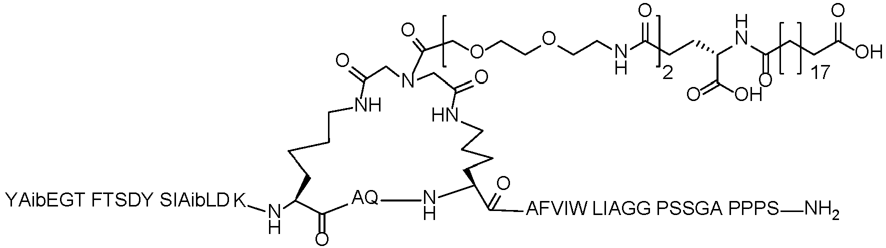 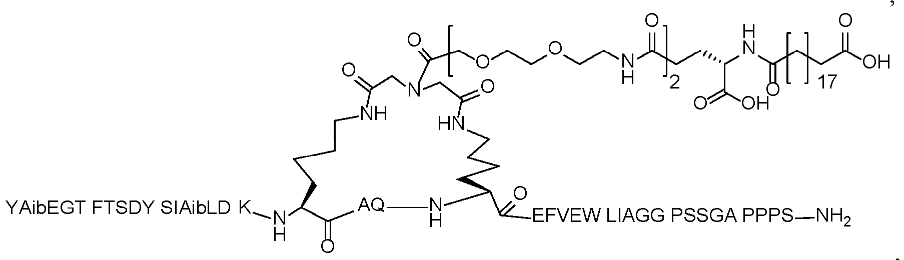 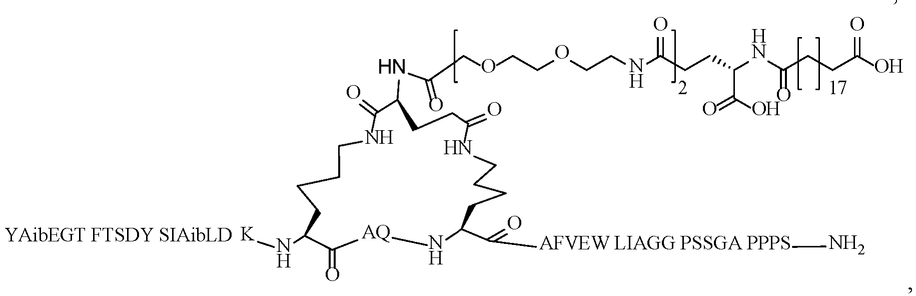 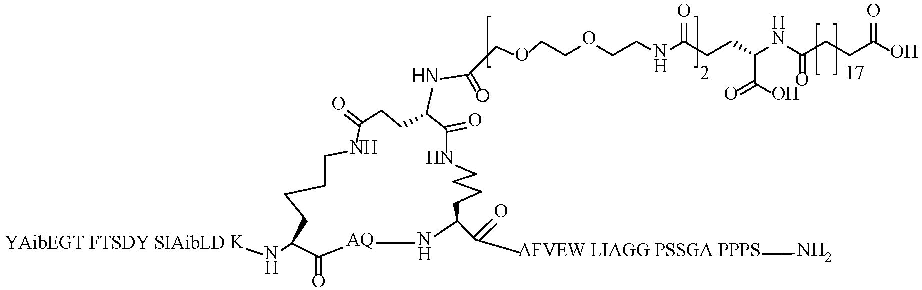 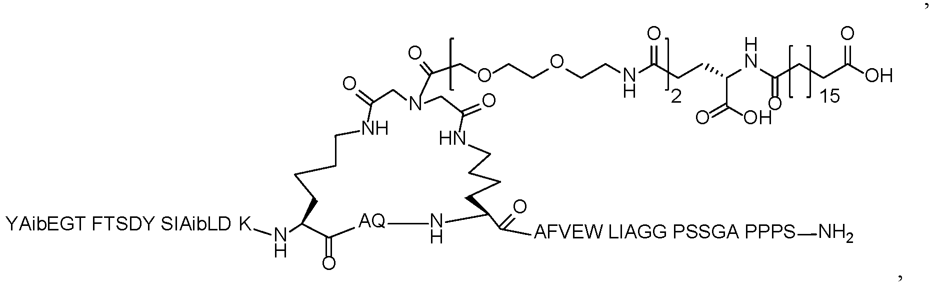 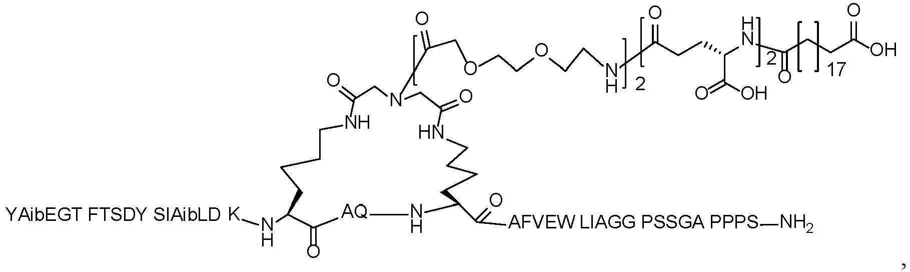 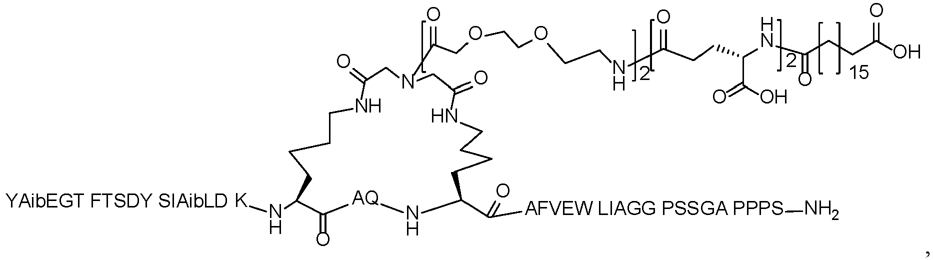 and

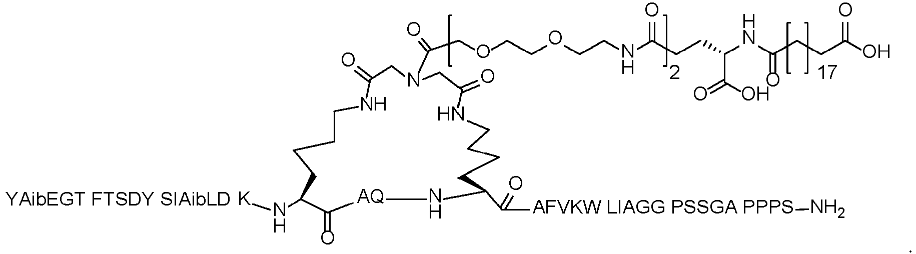

The present application further provides a use of a series of pharmaceutical compositions in the preparation of a drug for preventing and/or treating diabetes, obesity, and related complications (related diseases) thereof. The pharmaceutical compositions include a therapeutically effective amount of the above compound or a pharmaceutically acceptable salt thereof, as an active ingredient, and a pharmaceutically acceptable carrier.

Diabetes, a chronic disease with multiple pathological manifestations, is a metabolic endocrine disorder caused by absolute insulin deficiency or reduced biological effects of insulin, which is one of the specific indications of lipid metabolism disorder, circulatory disturbance, and/or glucose metabolism disorder within the body. In addition, diabetes is highly susceptible to various complications (related diseases), such as diabetic cardiovascular diseases, diabetic cerebrovascular diseases, retinopathy, nephropathy, cataracts, coronary artery diseases, diabetic neuropathy, diabetic foot, and peripheral neuropathy, wherein diabetic cardiovascular complications include microangiopathy in heart and great vessels, cardiomyopathy, cardiac autonomic neuropathy, etc; and diabetic cerebrovascular diseases include cerebral arteriosclerosis, ischemic cerebrovascular disease, cerebral hemorrhage, encephalatrophy, etc.

Obesity, another common metabolic syndrome caused by a combination of factors such as poor dietary habits, reduced activity, emotional factors, genetic factors, and others, is also one of the specific indications of lipid metabolism disorder, circulatory disturbance, and/or glucose metabolism disorder within the body. Meanwhile, obesity may increase the risk of liver diseases, hyperlipidemia, diabetes, hypertension, and other related diseases.

Diabetes, obesity, and related diseases thereof have serious impacts on human health and social economy.

In some embodiments of the present application, provided is a use of the above polypeptide compound or a pharmaceutically acceptable salt thereof or the above pharmaceutical composition in the preparation of a drug for treating diabetes and/or obesity.

In some embodiments of the present application, the above polypeptide compound or a pharmaceutically acceptable salt thereof or the above pharmaceutical composition is administered once every 3 days, once every 4 days, once a week, or once every two weeks.

### Technical effect

The polypeptide of the present application exhibits very strong agonistic activity on GLP-1R/GIPR. The polypeptide compound of the present application exhibits excellent pharmacokinetic property, plasma stability and extremely high plasma protein binding.

### Definition and Illustration

Unless otherwise indicated, the following terms and phrases used herein are intended to have the following meanings. A particular term or phrase should not be considered indefinite or unclear in the absence of a particular definition but should be understood in its common meaning. When a trade name is described herein, it is intended to refer to its corresponding commodity or its active ingredient.

The term "pharmaceutically acceptable" as used herein refers to the compounds, materials, compositions and/or dosage forms which are suitable for use in contact with human and animal tissues under the reliable medical judgment, without undue toxicity, irritation, allergic response or other problems or complications, and commensurate with a reasonable benefit/risk ratio.

The term "pharmaceutically acceptable salt" refers to a salt of the polypeptide compound of the present application, which is prepared from a compound having a specific substituent found in the present application and a relatively non-toxic acid or base. When the compound of the present application contains a relatively acidic functional group, a base addition salt can be obtained by contacting such compound with a sufficient amount of base in a pure solution or a suitable inert solvent. Pharmaceutically acceptable base addition salts include sodium, potassium, calcium, ammonium, organic amine, or magnesium salts or similar salts. When the compound of the present application contains a relatively basic functional group, an acid addition salt can be obtained by contacting such compound with a sufficient amount of acid in a pure solution or a suitable inert solvent. Examples of pharmaceutically acceptable acid addition salts include salts of inorganic acids including, such as, hydrochloric acid, hydrobromic acid, nitric acid, carbonic acid, bicarbonate, phosphoric acid, monohydrogen phosphate, dihydrogen phosphate, sulfuric acid, hydrosulfate, hydroiodic acid, phosphorous acid, etc.; and salts of organic acids including, such as, acetic acid, propionic acid, isobutyric acid, maleic acid, malonic acid, benzoic acid, succinic acid, octanedioic acid, fumaric acid, lactic acid, mandelic acid, phthalic acid, benzenesulfonic acid, p-toluenesulfonic acid, citric acid, tartaric acid, and methanesulfonic acid or other similar acids; and salts of amino acids (e.g., arginine, etc.), and salts of organic acids such as glucuronic acid or the like. Some specific compounds of the present application contain both basic and acidic functional groups and can thus be converted to either base or acid addition salt.

The pharmaceutically acceptable salts of the present application can be synthesized from parent compounds containing acid or base group through conventional chemical methods. Generally, such salts are prepared by reacting such compounds in form of free acid or base with a stoichiometric amount of an appropriate base or acid in water or an organic solvent or a mixture of both.

"Amino acids" refer to naturally occurring or synthesized amino acids, as well as amino acid analogues and amino acid mimetics that function similarly to naturally occurring amino acids. Naturally occurring amino acids are those encoded by genetic codes, as well as those modified thereafter, such as hydroxyproline, γ-carboxyglutamic acid, and O-phosphoserine. Amino acid analogues refer to compounds having the same basic chemical structures (e.g., α-carbon bound to hydrogen, carboxyl group, amino group, and R group) as those of naturally occurring amino acids, such as homoserine, norleucine, methionine sulfoxide, and methionine methylsulfonium. Such analogues may have modified R groups (e.g., norleucine) or modified peptide backbone, but retain the same basic chemical structures as those of naturally occurring amino acids. Amino acid mimetics refer to chemical compounds that have different chemical structures from those of conventional amino acids but perform a similar function to naturally occurring amino acids.

As used herein, A or Ala represents alanine with a structure of 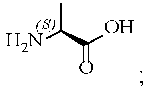 R or Arg represents arginine with a structure of 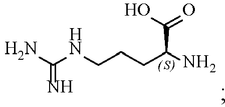 N or Asn represents asparagine with a structure of 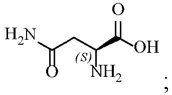 D or Asp represents aspartic acid with a structure of 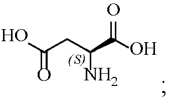 C or Cys represents cysteine with a structure of 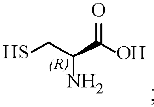 Q or Gln represents glutamine with a structure of 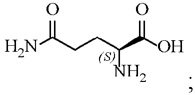 E or Glu represents glutamic acid with a structure of 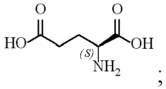 G or Gly represents glycine with a structure of 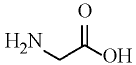 ; H or His represents histidine with a structure of 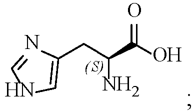 I or Ile represents isoleucine with a structure of 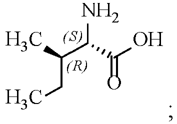 L or Leu represents leucine with a structure of 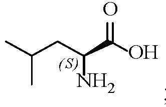 K or Lys represents lysine with a structure of 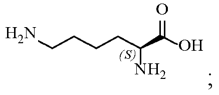 ; M or Met represents methionine with a structure of 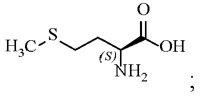 F or Phe represents phenylalanine with a structure of 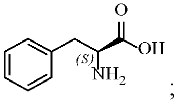 P or Pro represents proline with a structure of 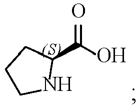 S or Ser represents serine with a structure of 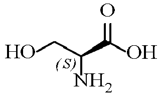 ; T or Thr represents threonine with a structure of 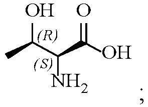 W or Trp represents tryptophan with a structure of 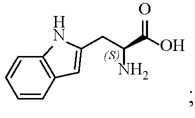 Y or Tyr represents tyrosine with a structure of 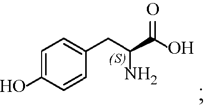 V or Val represents valine with a structure of

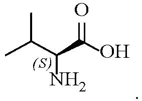

The term "treatment" includes inhibiting, alleviating, stopping, or reversing the progression or severity of an existing symptom or disease.

Unless otherwise indicated, the term "isomer" is intended to include geometrical isomers, cis-trans isomers, stereoisomers, enantiomers, optical isomers, diastereomers, and tautomers.

The compound of the present application may exist in specific geometric or stereoisomeric forms. The present application contemplates all such compounds, including cis and trans isomers, (-)- and (+)-enantiomers, (*R*)- and (*S*)-enantiomers, diastereomers, (*D*)-isomers, (*L*)-isomers, and their racemic mixtures and other mixtures, such as enantiomerically or diastereomerically enriched mixtures, all of which are subject to the scope of the present application. Additional asymmetric carbon atoms may be present in substituents such as alkyl groups. All these isomers and mixtures thereof are all included within the scope of the present application.

Unless otherwise indicated, the term "enantiomers" or "optical isomers" refers to stereoisomers that are mirror images of each other.

Unless otherwise indicated, the term "cis-trans isomers" or "geometrical isomers" refers to isomers that result from the restricted rotation around double bonds or single bonds of cyclic carbon atoms.

Unless otherwise indicated, the term "diastereomers" refers to stereoisomers that have two or more chiral centers and exhibit non-mirror-image relationships between molecules.

Unless otherwise indicated, "(+)" represents dextro, "(-)" represents levo, "(±)" represents racemic.

Unless otherwise indicated, a wedge solid line bond ( 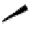 ) and a wedge dotted line bond ( 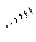 ) are used to represent the absolute configuration of a stereocenter, a straight solid line bond ( 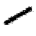 ) and a straight dotted line bond () are used to represent the relative configuration of a stereocenter; and a wavy line ( 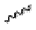 ) may be used to represent the wedge solid line bond ( 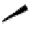 ) or the wedge dotted line bond ( 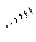 ), or a wavy line ( 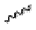 ) may be used to represent the straight solid line bond ( 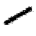 ) or the straight dotted line bond ( 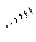 ).

Unless otherwise indicated, the term "enrich in one isomer", "isomerically enriched", "enrich in one enantiomer" or "enantiomerically enriched" means that the content of the one isomer or enantiomer therein is less than 100% but greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise indicated, the term "isomerically excess" or "enantiomerically excess" refers to the difference between the relative percentages of two isomers or two enantiomers. For example, the content of one isomer or enantiomer is 90%, and the content of the other isomer or enantiomer is 10%, then the isomer or enantiomer is 80% excess (ee value).

Optically active (*R*)- and (*S*)-isomers as well as *D* and *L*-isomers may be prepared by chiral synthesis or using chiral reagents or other conventional techniques. To obtain one enantiomer of a compound of the present application, it can be prepared through asymmetric synthesis or derivatization with a chiral auxiliary agent. The resulting diastereomeric mixture is then separated and the auxiliary group is cleaved to provide the pure desired enantiomer. Alternatively, when the molecule contains a basic functional group (such as an amino group) or an acidic functional group (such as a carboxyl group), salts of diastereomers may be formed by reacting with an appropriate optically active acid or base. The diastereomers may then be resolved through conventional methods known in the art, followed by recovery of the pure enantiomers. In addition, the separation of enantiomers and diastereomers is generally achieved by using chromatography in which chiral stationary phases are employed, optionally in combination with chemical derivatization (for example, generation of carbamates from amines).

The compounds of the present application may contain unnatural proportions of atomic isotopes on one or more of the atoms that constitute the compounds. For example, the compounds may be labeled with radioactive isotopes, e.g., tritium (³H), iodine-125 (¹²⁵ I) or C-14 (¹⁴C). Further for example, hydrogen may be replaced by deuterium to form deuterated drugs. The bond between deuterium and carbon is stronger than that between ordinary hydrogen and carbon, and compared with non-deuterated drugs, the deuterated drugs have the advantages of reduced toxic and side effects, increased drug stability, enhanced efficacy, and extended biological half-life of drugs. All variations in isotopic composition of the compounds of the present application, whether radioactive or not, are included within the scope of the application.

When the linking group listed does not indicate its linking direction, its linking direction is arbitrary. For example, the linking group L in 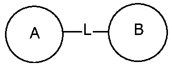 is -M-W-, and -M-W- may either link the ring A and ring B in the same direction as the reading order from left to right to form 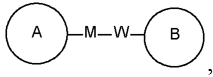 or link the ring A and ring B in the opposite direction to the reading order from left to right to form 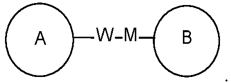 Combinations of the linking groups, substituents and/or variants thereof are permissible only if such combinations result in stable compounds.

Unless otherwise specified, when a group has one or more linkable sites, any one or more of the sites of the group may be linked to other groups through chemical bonds. If the connection mode of the chemical bond is non-directional and there are hydrogen atoms at the connecting site, when the chemical bond is connected, the number of hydrogen atoms at that site will decrease correspondingly with the number of connected chemical bonds, forming a group of the corresponding valence. The chemical bonds between the site and other groups may be represented by a straight solid line bond ( 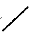 ), a straight dotted line bond ( 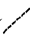 ), or a wavy line ( 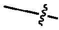 ). For example, the straight solid line bond in -OCH₃ indicates that the group is connected to other group through the oxygen atom in that group; the straight dotted line bond in 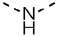 indicates that the group is connected to other groups at the two ends of the nitrogen atom in that group; and the wavy line in 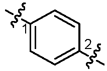 indicates that the phenyl group is connected to other groups through the carbon atoms at positions 1 and 2 of the phenyl group.

Unless otherwise specified, the term "C₁₋₃ alkyl" is used to represent a linear or branched saturated hydrocarbon group composed of 1 to 3 carbon atoms. The C₁₋₃ alkyl includes C₁₋₂ and C₂₋₃ alkyl, etc., and it may be monovalent (e.g., methyl), divalent (e.g., methylene), or multivalent (e.g., methylidyne). Examples of C₁₋₃ alkyl include, but not limited to, methyl (Me), ethyl (Et), propyl (including n-propyl and isopropyl), etc.

The structure of the compounds of the present application may be determined by conventional methods known to those skilled in the art. If the present application involves the absolute configuration of the compounds, the absolute configuration may be confirmed using conventional techniques in the art. For example, single crystal X-ray diffraction (SXRD) may be used, in which a Bruker D8 venture diffractometer is used to collect diffraction intensity data of the cultured single crystal with CuKα radiation as the light source and in a scanning mode of ϕ/ω scanning, and after collecting relevant data, the crystal structure may be further resolved by a direct method (Shelxs97), thus confirming the absolute configuration.

The compounds of the present application may be prepared by a variety of synthetic methods well known to those skilled in the art, including the specific embodiments listed below, the embodiments resulted from the combination with other chemical synthetic methods, and equivalent alternatives well known to those skilled in the art. The preferred embodiments include, but not limited to, the examples of the present application.

The solvents used herein are commercially available.

The following abbreviations are used herein: aq represents aqueous, eq represents equivalent or equal amount; DCM represents dichloromethane; PE represents petroleum ether; DMSO represents dimethylsulfoxide; MeOH represents methanol; BOC represents tert-butoxycarbonyl, an amine-protecting group; r.t. represents room temperature; O/N represents overnight; THF represents tetrahydrofuran; Boc₂O represents di-tert-butyl dicarbonate; TFA represents trifluoroacetic acid; DIEA represents diisopropylethylamine; DMF represents N, N-dimethylformamide; HBTU represents benzotriazole-N, N, N', N'-tetramethyl-uronium-hexafluorophosphate; HOBT represents 1-hydroxybenzotriazole; HOAT represents 1-hydroxy-7-azabenzotriazole; DIC represents N, N'-diisopropylcarbodiimide; DBU represents 1, 8-diazabicyclo[5.4.0]undec-7-ene; PhSiH₃ represents phenylsilane; and Pd(PPh₃)₄ represents tetrakis(triphenylphosphine)palladium.

### DETAILED DESCRIPTION

The present application will be described in detail through examples below, but it does not imply that any unfavorable limitation is imposed on the present application. Although the present application has been described in detail herein where specific embodiments are also disclosed, it is apparent to those skilled in the art that various change and modification may be made to the specific embodiments of the present application without departing from the essence and scope of the present application.

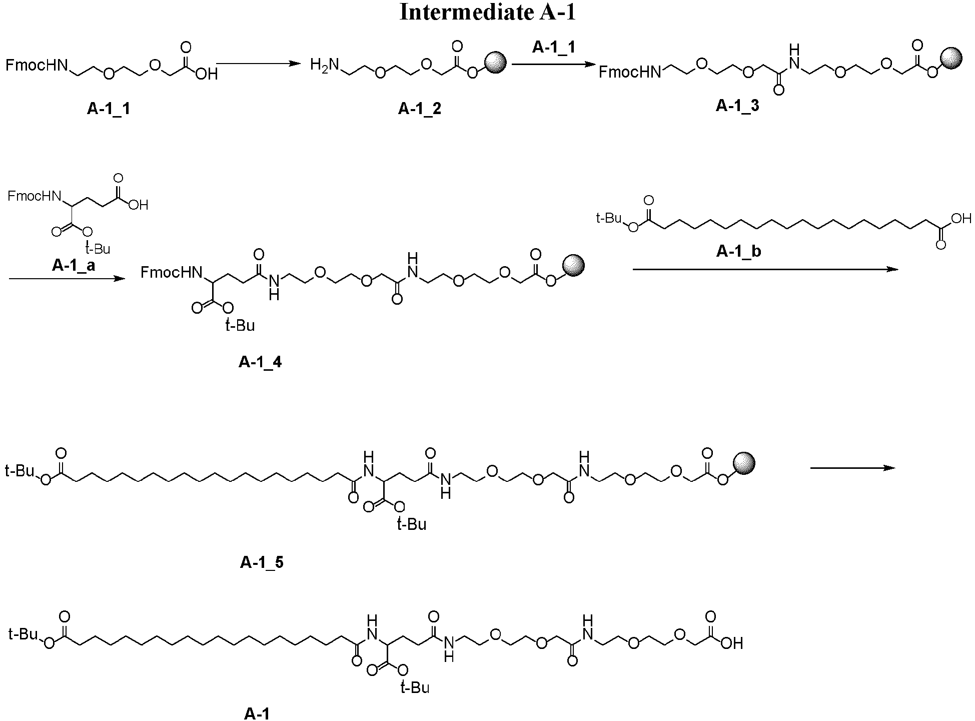

### Step 1: Resin filling

**1.1** 4 g of chloro-(o-chlorophenyl)-diphenylmethane resin (degree of substitution, S = 1.00 mmol/g) and 1.54 g of **A-1_1** were weighed into a reaction column, into which were further added DCM (25 mL) and then 3 mL of N, N-diisopropylethylamine, and the reaction column was aerated with nitrogen for 2 h. After then, 4 mL of MeOH was added, and the reaction column was continually aerated with nitrogen for 30 minutes. The waste was drained until no liquid flew out. The reaction column was washed with DMF (50 mL) for 5 times, each time for 1 min, and the waste was drained until no liquid flew out.

**1.2** 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.

### Step 2: Coupling of amino acids

### 2.1 Coupling of A-1_1

a) **A-1_1** (3.0 eq) was weighed and added into the resin, DIEA (6.00 eq) plus 20 mL of DMF were supplemented into the reaction column, which was then aerated with nitrogen and added with HBTU (2.85 eq) after the amino acids were dissolved. Nitrogen was adjusted to make the resin bulge evenly.
b) The reaction was conducted at 25°C for 20 min. Ninhydrin was used for detection, with the resin appearing colorless and transparent.
c) The reaction solution was drawn off from the reaction column which was then washed with DMF for 5 times, each time for 1 min, and the waste was drained until no liquid flew out.

### 2.2 Coupling of A-1_a

a) 20% piperidine in DMF (50 mL) was added into the reaction column which was then aerated with nitrogen for 20 min. The waste was drained until no liquid flew out. Ninhydrin was used for detection, with the resin appearing blue.
b) A-1_a (3.0 eq) was weighed and added into the resin, DIEA (3.00 eq) plus 20 mL of DMF were supplemented into the reaction column, which was then aerated with nitrogen and added with HBTU (2.85 eq) after the amino acids were dissolved. Nitrogen was adjusted to make the resin bulge evenly.
c) The reaction was conducted at 25°C for 0.5 h. Ninhydrin was used for detection, with the resin appearing colorless and transparent.
d) The reaction solution was drawn off from the reaction column which was then washed with DMF (50 mL) for 5 times, each time for 1 min, and the waste was drained until no liquid flew out.

### 2.3 Coupling of A-1_b

a) 20% piperidine in DMF (50mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
b) **A-1_b** (3.0 eq) was weighed and added into the resin, DIEA (6.00 eq) plus 20 mL of DMF were supplemented into the reaction column, which was then aerated with nitrogen and added with HBTU (2.85 eq) after the amino acids were dissolved. Nitrogen was adjusted to make the resin bulge evenly.
c) The reaction was conducted at 25°C for 0.5 h. Ninhydrin was used for detection, with the resin appearing colorless and transparent.
d) The reaction solution was drawn off from the reaction column which was then washed with DMF for 5 times, each time for 1 min, and the waste was drained until no liquid flew out.

### Step 3: Cutting and crude peptide drying

3.1 A cleaving solution was formulated following the volumes below:

| **Reagents** | **Proportion** |
|---|---|
| **Hexafluoroisopropanol (HFIP)** | 20 |
| **DCM** | 80 |

3.2 60 mL of the formulated cleaving solution was poured into a reactor containing the resin with the peptide after being dried. The reactor was aerated for 20 min, the resulted solution was filtered, and the filtrate was added into a flask. The operation was repeated twice, and the collected solution in the two operations was spin-dried to obtain **A-1.**

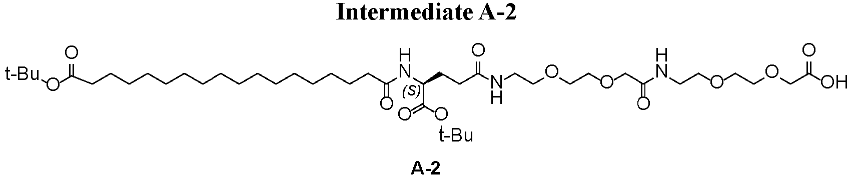

Intermediate **A-2** was obtained with reference to the synthesis of Intermediate **A-1.**

### Step 1: Resin filling

**1.1** 4 g of chloro-(o-chlorophenyl)-diphenylmethane resin (degree of substitution, S = 1.00 mmol/g) and 1.54 g of Fmoc-AEEA-OH were weighed into a reaction column, into which were further added DCM (25 mL) and then 3 mL of DIEA, and the reaction column was aerated with nitrogen for 2 h. After then, 4 mL of MeOH was added, and the reaction column was continually aerated with nitrogen for 30 minutes. The waste was drained until no liquid flew out. The reaction column was washed with DMF (50 mL) for 5 times, each time for 1 min, and the waste was drained until no liquid flew out.

**1.2** 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.

### Step 2: Coupling of amino acids

### 2.1 Coupling of Fmoc-AEEA-OH

1. Fmoc-AEEA-OH (3.00 eq) was weighed and added into the resin, DIEA (6.00 eq) plus 20 mL of DMF were supplemented into the reaction column, which was then aerated with nitrogen and added with HBTU (2.85 eq) after the amino acids were dissolved. Nitrogen was adjusted to make the resin bulge evenly.
2. The reaction was conducted at 25°C for 20 min. Ninhydrin was used for detection, with the resin appearing colorless and transparent.
3. The reaction solution was drawn off from the reaction column which was then washed with DMF for 5 times, each time for 1 min, and the waste was drained until no liquid flew out.

### 2.2 Coupling of Fmoc-Glu-OtBu

1. 20% piperidine in DMF (50 mL) was added into the reaction column which was then aerated with nitrogen for 20 min. The waste was drained until no liquid flew out. Ninhydrin was used for detection, with the resin appearing blue.
2. Fmoc-Glu-OtBu (3.00 eq) was weighed and added into the resin, DIEA (3.00 eq) plus 20 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF (50 mL) for 5 times, each time for 1 min, and the waste was drained until no liquid flew out.

### 2.3 Coupling of 20-(tBu)-eicosandioic acid

1. 20% piperidine in DMF (50mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. 20-(tBu)-eicosandioic acid (3.00 eq) was weighed and added into the resin, DIEA (6.00 eq) plus 20 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times, each time for 1 min, and the waste was drained until no liquid flew out.

### Step 3: Cutting and crude peptide drying

3.1 A cleaving solution was formulated following the volumes below:

| **Reagents** | **Proportion** |
|---|---|
| **Hexafluoroisopropanol (HFIP)** | 20 |
| **DCM** | 80 |

3.2 The operation was repeated twice, and the collected cleaving solutions in the two operations were spin-dried to obtain **A-2.**

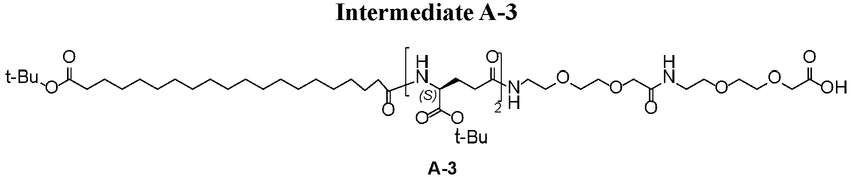

Intermediate **A-3** was obtained with reference to the synthesis of Intermediate **A-1.**

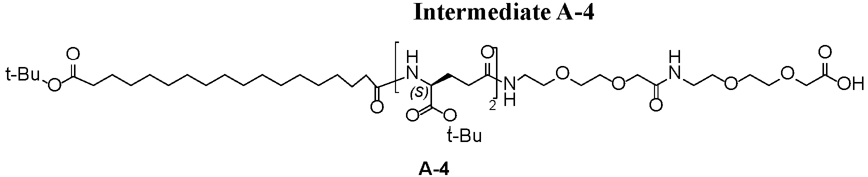

Intermediate **A-4** was obtained with reference to the synthesis of Intermediate **A-1.** 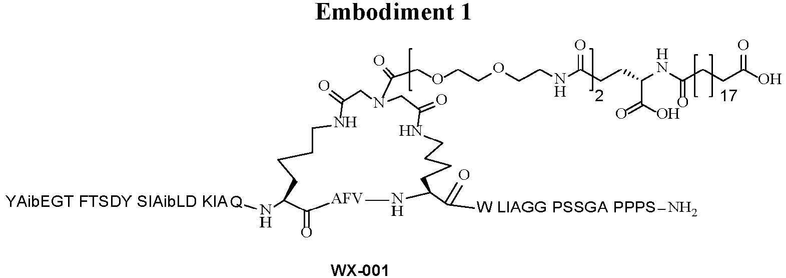
**Step 1:** 1.34 g of 4-(2',4'-dimethoxyphenyl-fluorenemethoxycarbonyl-aminomethyl)-phenoxyacetamido-methylbenzhydrylamine resin (degree of substitution, Sub = 0.3 mmol/g) was weighed and added into a reaction column, into which was further added DMF (50 mL), and the reaction column was aerated with nitrogen for 2 h. After then, the waste was drained until no liquid flew out. The reaction column was washed with DMF (50 mL) for 5 times, each time for 1 min, and the waste was drained until no liquid flew out.
**Step 2:** 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.

### Step 3: Coupling of amino acids

### 3.1 Coupling of Fmoc-Ser(tBu)-OH

1. Fmoc-Ser(tBu)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.2 Coupling of Fmoc-Pro-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.3 Coupling of Fmoc-Pro-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Chloranil was used for detection, with the resin appearing colorless and transparent.
4. The reaction solution was drawn off from the reaction column which was then washed with DMF for 5 times, each time for 1 min, and the waste was drained until no liquid flew out.

### 3.4 Coupling of Fmoc-Pro-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.5 Coupling of Fmoc-Ala-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Ala-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.6 Coupling of Fmoc-Gly-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gly-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.7 Coupling of Fmoc-Ser(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.8 Coupling of Fmoc-Ser(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.9 Coupling of Fmoc-Pro-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.10 Coupling of Fmoc-Gly-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Gly-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.11 Coupling of Fmoc-Gly-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gly-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.12 Coupling of Fmoc-Ala-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.13 Coupling of Fmoc-Ile-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ile-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.14 Coupling of Fmoc-Leu-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Leu-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.15 Coupling of Fmoc-Trp(Boc)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Trp(Boc)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.16 Coupling of Fmoc-Lys(Dde)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Dde)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.17 Coupling of Fmoc-Val-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Val-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.18 Coupling of Fmoc-Phe-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Phe-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.19 Coupling of Fmoc-Ala-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.20 Coupling of Fmoc-Lys(Alloc)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Alloc)-OH (2.0 eq) was weighed and added into the resin, HOBT (2.00 eq) plus 10 mL of DMF were supplemented into the reaction column, which was then aerated with nitrogen and added with DIC (2.00 eq) after the amino acids and HOBT were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.21 Coupling of Fmoc-Gln(Trt)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gln(Trt)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.22 Coupling of Fmoc-Ala-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.23 Coupling of Fmoc-Ile-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ile-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.24 Coupling of Fmoc-Lys(Boc)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Boc)-OH (2.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.25 Coupling of Fmoc-Asp(OtBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Asp(OtBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.26 Coupling of Fmoc-Leu-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Leu-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.27 Coupling of Fmoc-Aib-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min and the waste was drained until no liquid flew out. Ninhydrin was used for detection, with the resin appearing blue.
2. Fmoc-Aib-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was conducted at 25°C for 2 h. Ninhydrin was used for detection, with the resin appearing blue.
4. The reaction solution was drawn off from the reaction column which was then washed with DMF for 5 times (50 mL each time), each time for 1 min, and the waste was drained until no liquid flew out.

### 3.28 Coupling of Fmoc-Ile-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ile-OH (6.0 eq) was weighed and added into the resin, HOAT (6.00 eq) plus 10 mL of DMF were supplemented into the reaction column, which was then aerated with nitrogen and added with DIC (6.00 eq) after the amino acids and HOAT were dissolved. Nitrogen was adjusted to make the resin bulge evenly.
3. The reaction was conducted at 25°C for 1 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.29 Coupling of Fmoc-Ser(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.30 Coupling of Fmoc-Tyr(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Tyr(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.31 Coupling of Fmoc-Asp(OtBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Asp(OtBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.32 Coupling of Fmoc-Ser(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.33 Coupling of Fmoc-Thr(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Thr(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.34 Coupling of Fmoc-Phe-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Phe-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.35 Coupling of Fmoc-Thr(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Thr(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.36 Coupling of Fmoc-Gly-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gly-OH (3.0 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.37 Coupling of Fmoc-Glu(OtBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Glu(OtBu)-OH (6.0 eq) was weighed and added into the resin, HOBT (6.00 eq) plus 10 mL of DMF were supplemented into the reaction column, which was then aerated with nitrogen and added with DIC (6.00 eq) after the amino acids and HOBT were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.38 Coupling of Fmoc-Aib-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Aib-OH (3.0 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C overnight. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.39 Coupling of Boc-Tyr(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Boc-Tyr(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was conducted at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.40 Removal of Alloc

1. PhSiH₃ (10.0 eq) and DCM (10 mL) were added into the reaction column, which was then aerated with nitrogen and added with Pd(PPh₃)₄ (0.1 eq), then aerated with nitrogen for 20 min. The reaction was carried out twice, and the waste was drained until no liquid flew out.
2. The reaction column was washed with DMF for 5 times (50 mL each time), each time for 1 min, and the waste was drained until no liquid flew out.

### 3.41 Coupling of Fmoc-Ida-OH

1. Fmoc-Ida-OH (4.0 eq) was weighed and added into the resin, DIEA (8.00 eq) plus 10 mL of DMF were supplemented into the reaction column, which was then aerated with nitrogen and added with HBTU (3.80 eq) after the amino acids were dissolved. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.42 Coupling of Intermediate A-1

1. 10% DBU in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. **Intermediate A-1** (1.50 eq) was weighed and added into the resin, DIEA (3.00 eq) plus 10 mL of DMF were supplemented into the reaction column, which was then aerated with nitrogen and added with HBTU (1.45 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.43 Removal of Dde

1. 3% hydrazine in DMF (50 mL) was added into the reaction column which was then aerated with nitrogen for 15 min, and the waste was drained. The reaction column was washed with DMF (50 mL) for 5 times, each time for 1 min, and the waste was drained until no liquid flew out. Ninhydrin was used for detection, with the resin appearing blue.

### 3.44 Amide ring closing

1. DIEA (3.0 eq) in DMF was added into the resin, then a solution of HATU (1.5 eq) in DMF was dropwise added into the reaction column slowly, which was then aerated with nitrogen. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.
4. The resin was shrunk with MeOH (50 mL) for 3 min each time. The waste was drained until no liquid flew out. The resin was poured out and dried for further use.

### Step 4: Cutting and crude peptide drying

4.1 A cleaving solution was formulated following the volumes below:

| **Reagents** | **Proportion** |
|---|---|
| **Trifluoroacetic acid** | 92.5 |
| **Triisopropylsilane** | 2.5 |
| **H₂O** | 2.5 |
| **3-mercaptopropionic acid** | 2.5 |

4.2 The dried resin with the peptide was added into the formulated cleaving solution, and the resulted solution was shaken on a shaker for 2.5 h, and filtered. The filtrate was added into 10 times the volume of ice-cold isopropyl ether, the resulted mixture was centrifuged, and the centrifuged deposit was washed for 5 times with isopropyl ether. The washed product was then dried in vacuum for 2 h to get a crude peptide, which was purified to obtain a polypeptide compound **WX-001.** The molecular weight of the polypeptide was confirmed by ESI-MS, with a calculated value of [M+4H]/4 as 1228.6 and a detected value of 1228.7.

### Embodiment 2

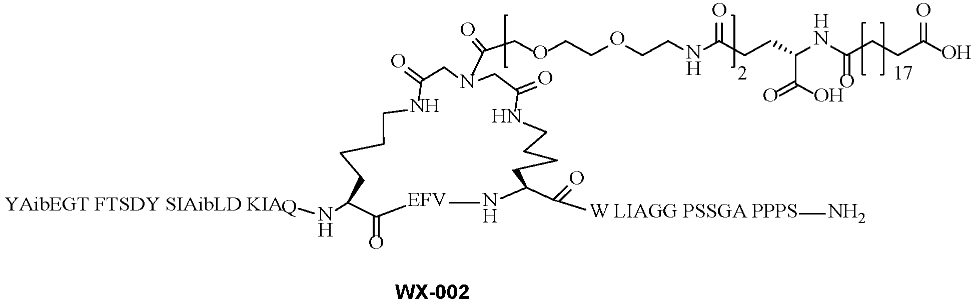

With reference to the synthesis of **WX-001,** a polypeptide **WX-002** was obtained. The molecular weight of the polypeptide was confirmed by ESI-MS, with a calculated value of [M+4H]/4 as 1243.1 and a detected value of 1242.8.

### Embodiment 3

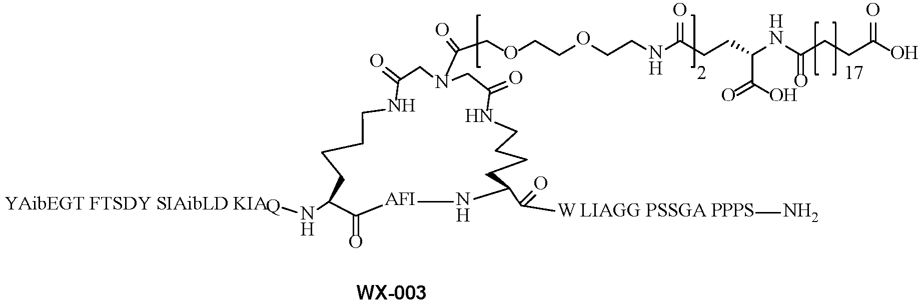

With reference to the synthesis of **WX-001,** a polypeptide **WX-003** was obtained. The molecular weight of the polypeptide was confirmed by ESI-MS, with a calculated value of [M+4H]/4 as 1232.1 and a detected value of 1232.2.

### Embodiment 4

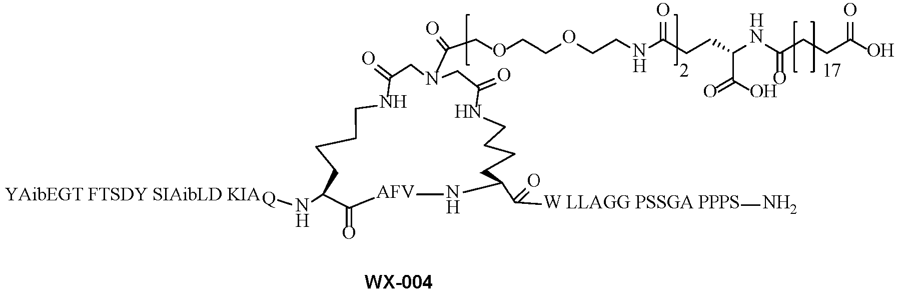

With reference to the synthesis of **WX-001,** a polypeptide **WX-004** was obtained. The molecular weight of the polypeptide was confirmed by ESI-MS, with a calculated value of [M+4H]/4 as 1228.6 and a detected value of 1228.2.

### Embodiment 5

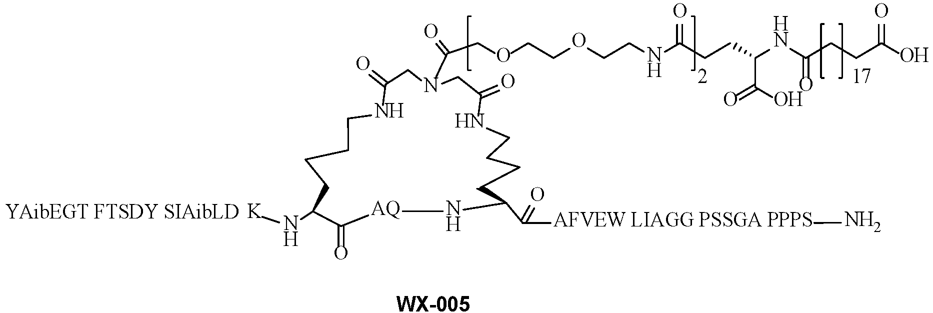

**Step 1:** 1.34 g of 4-(2', 4'-dimethoxyphenyl-fluorenemethoxycarbonyl-aminomethyl)-phenoxyacetamido-methylbenzhydrylamine resin (degree of substitution, Sub = 0.3 mmol/g) was weighed and added into a reaction column, into which was further added DMF (50 mL), and the reaction column was aerated with nitrogen for 2 h. After then, the waste was drained until no liquid flew out. The reaction column was washed with DMF (50 mL) for 5 times, each time for 1 min, and the waste was drained until no liquid flew out.
**Step 2:** 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.

### Step 3: Coupling of amino acids

### 3.1 Coupling of Fmoc-Ser(tBu)-OH

1. Fmoc-Ser(tBu)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.2 Coupling of Fmoc-Pro-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.
**5. Step 3.2 was similarly repeated to perform the coupling of the following amino acids.**

| **No.** | **Raw materials** | **Coupling reagents** |
|---|---|---|
| 3.3 | Fmoc-Pro-OH (3.00 eq) | HBTU (2.85 eq) and DIEA (6.00 eq) |
| 3.4 | Fmoc-Pro-OH (3.00 eq) | HBTU (2.85 eq) and DIEA (6.00 eq) |
| 3.5 | Fmoc-Ala-OH (3.00 eq) | HBTU (2.85 eq) and DIEA (6.00 eq) |
| 3.6 | Fmoc-Gly-OH (3.00 eq) | HBTU (2.85 eq) and DIEA (6.00 eq) |
| 3.7 | Fmoc-Ser(tBu)-OH (3.00 eq) | HBTU (2.85 eq) and DIEA (6.00 eq) |
| 3.8 | Fmoc-Ser(tBu)-OH (3.00 eq) | HBTU (2.85 eq) and DIEA (6.00 eq) |
| 3.9 | Fmoc-Pro-OH (3.00 eq) | HBTU (2.85 eq) and DIEA (6.00 eq) |
| 3.10 | Fmoc-Gly-OH (3.00 eq) | HBTU (2.85 eq) and DIEA (6.00 eq) |
| 3.11 | Fmoc-Gly-OH (3.00 eq) | HBTU (2.85 eq) and DIEA (6.00 eq) |
| 3.12 | Fmoc-Ala-OH (3.00 eq) | HBTU (2.85 eq) and DIEA (6.00 eq) |
| 3.13 | Fmoc-Ile-OH (3.00 eq) | HBTU (2.85 eq) and DIEA (6.00 eq) |
| 3.14 | Fmoc-Leu-OH (3.00 eq) | HBTU (2.85 eq) and DIEA (6.00 eq) |
| 3.15 | Fmoc-Trp(Boc)-OH (3.00 eq) | HBTU (2.85 eq) and DIEA (6.00 eq) |
| 3.16 | Fmoc-Glu(tBu)-OH (2.00 eq) | HBTU (1.90 eq) and DIEA (4.00 eq) |
| 3.17 | Fmoc-Val-OH (3.00 eq) | HBTU (2.85 eq) and DIEA (6.00 eq) |
| 3.18 | Fmoc-Phe-OH (3.00 eq) | HBTU (2.85 eq) and DIEA (6.00 eq) |
| 3.19 | Fmoc-Ala-OH (3.00 eq) | HBTU (2.85 eq) and DIEA (6.00 eq) |
| 3.20 | Fmoc-Lys(Dde)-OH(3.00 eq) | HOBT (2.00 eq) and DIC (2.00 eq) |
| 3.21 | Fmoc-Gln(Trt)-OH (3.00 eq) | HBTU (2.85 eq) and DIEA (6.00 eq) |
| 3.22 | Fmoc-Ala-OH (3.00 eq) | HBTU (2.85 eq) and DIEA (6.00 eq) |
| 3.23 | Fmoc-Lys(Alloc)-OH (2.00 eq) | HBTU (2.85 eq) and DIEA (6.00 eq) |
| 3.24 | Fmoc-Lys(Boc)-OH (3.00 eq) | HBTU (2.85 eq) and DIEA (6.00 eq) |
| 3.25 | Fmoc-Asp(tBu)-OH (6.00 eq) | HATU (5.70 eq) and DIEA (12.0 eq) |
| 3.26 | Fmoc-Leu-OH (6.00 eq) | HATU (5.70 eq) and DIEA (12.0 eq) |
| 3.27 | Fmoc-Aib-OH (6.00 eq) | HATU (5.70 eq) and DIEA (12.0 eq) |
| 3.28 | Fmoc-Ile-OH (6.00 eq) | HOAT (6.00 eq) and DIC (6.00 eq) |
| 3.29 | Fmoc-Ser(tBu)-OH (6.00 eq) | HATU (5.70 eq) and DIEA (12.0 eq) |
| 3.30 | Fmoc-Tyr(tBu)-OH (6.00 eq) | HATU (5.70 eq) and DIEA (12.0 eq) |
| 3.31 | Fmoc-Asp(tBu)-OH (6.00 eq) | HATU (5.70 eq) and DIEA (12.0 eq) |
| 3.32 | Fmoc-Ser(tBu)-OH (6.00 eq) | HATU (5.70 eq) and DIEA (12.0 eq) |
| 3.33 | Fmoc-Thr(tBu)-OH (6.00 eq) | HATU (5.70 eq) and DIEA (12.0 eq) |
| 3.34 | Fmoc-Phe-OH (6.00 eq) | HATU (5.70 eq) and DIEA (12.0 eq) |
| 3.35 | Fmoc-Thr(tBu)-OH (6.00 eq) | HATU (5.70 eq) and DIEA (12.0 eq) |
| 3.36 | Fmoc-Gly-OH (6.00 eq) | HATU (5.70 eq) and DIEA (12.0 eq) |
| 3.37 | Fmoc-Glu(tBu)-OH (6.00 eq) | HOBT (6.00 eq) and DIC (6.00 eq) |
| 3.38 | Fmoc-Aib-OH (3.00 eq) | HATU (2.85 eq) and DIEA (6.00 eq) |
| 3.39 | Boc-Tyr(tBu)-OH (3.00 eq) | HATU (2.85 eq) and DIEA (6.00 eq) |

### 3.40 Removal of Alloc

1. PhSiH₃ (10.0 eq) and DCM (10 mL) were added into the reaction column, which was then aerated with nitrogen and added with Pd(PPh₃)₄ (0.1 eq), then aerated with nitrogen for 20 min. The reaction was carried out twice, and the waste was drained until no liquid flew out.
2. The reaction column was washed with DMF for 5 times (50 mL each time), each time for 1 min, and the waste was drained until no liquid flew out.

### 3.41 Coupling of Fmoc-Ida-OH

1. Fmoc-Ida-OH (4.0 eq) was weighed and added into the resin, DIEA (8.00 eq) plus 10 mL of DMF were supplemented into the reaction column, which was then aerated with nitrogen and added with HBTU (3.80 eq) after the amino acids were dissolved. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.42 Coupling of Intermediate A-1

1. 10% DBU in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. **Intermediate A-1** (1.50 eq) was weighed and added into the resin, DIEA (3.00 eq) plus 10 mL of DMF were supplemented into the reaction column, which was then aerated with nitrogen and added with HBTU (1.45 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.43 Removal of Dde

1. 3% hydrazine in DMF (50 mL) was added into the reaction column which was then aerated with nitrogen for 15 min, and the waste was drained. The reaction column was washed with DMF (50 mL) for 5 times, each time for 1 min, and the waste was drained until no liquid flew out. Ninhydrin was used for detection, with the resin appearing blue.

### 3.44 Amide ring closing

1. DIEA (3.0 eq) in DMF was added into the resin, then a solution of HATU (1.5 eq) in DMF was dropwise added into the reaction column slowly, which was then aerated with nitrogen. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.
4. The resin was shrunk with MeOH (50 mL) for 3 min each time. The waste was drained until no liquid flew out. The resin was poured out and dried for further use.

### Step 4: Cutting and crude peptide drying

4.1 A cleaving solution was formulated following the volumes below:

| **Reagents** | **Proportion** |
|---|---|
| **Trifluoroacetic acid** | 92.5 |
| **Triisopropylsilane** | 2.5 |
| **H₂O** | 2.5 |
| **3-mercaptopropionic acid** | 2.5 |

4.2 The dried resin with the peptide was added into the formulated cleaving solution, and the resulted solution was shaken on a shaker for 2.5 h, and filtered. The filtrate was added into 10 times the volume of ice-cold isopropyl ether, the resulted mixture was centrifuged, and the centrifuged deposit was washed for 5 times with isopropyl ether. The washed product was then dried in vacuum for 2 h to get a crude peptide, which was purified to obtain a polypeptide **WX-005.** The molecular weight of the polypeptide was confirmed by ESI-MS, with a calculated value of [M+4H]/4 as 1232.6 and a detected value of 1232.5.

### Embodiment 6

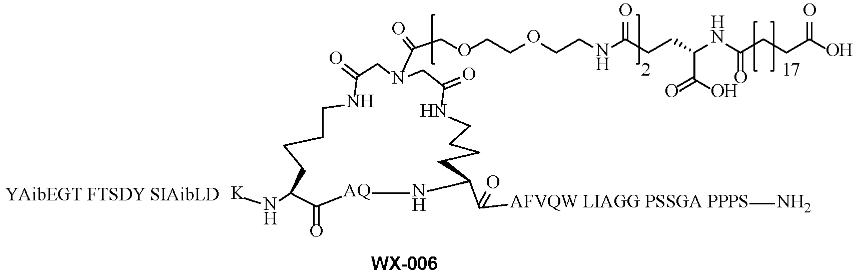

With reference to the synthesis of **WX-001,** a polypeptide **WX-006** was obtained. The molecular weight of the polypeptide was confirmed by ESI-MS, with a calculated value of [M+4H]/4 as 1232.4 and a detected value of 1232.4.

### Embodiment 7

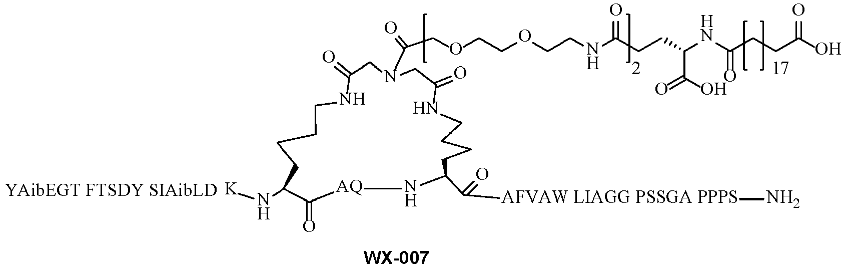

With reference to the synthesis of **WX-001,** a polypeptide **WX-007** was obtained. The molecular weight of the polypeptide was confirmed by ESI-MS, with a calculated value of [M+4H]/4 as 1218.1 and a detected value of 1218.3.

### Embodiment 8

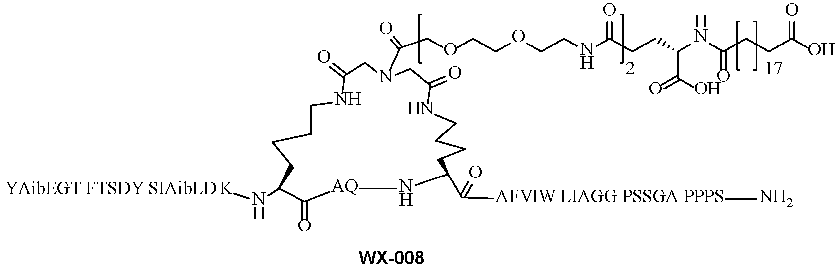

With reference to the synthesis of **WX-001,** a polypeptide **WX-008** was obtained. The molecular weight of the polypeptide was confirmed by ESI-MS, with a calculated value of [M+4H]/4 as 1228.6 and a detected value of 1228.6.

### Embodiment 9

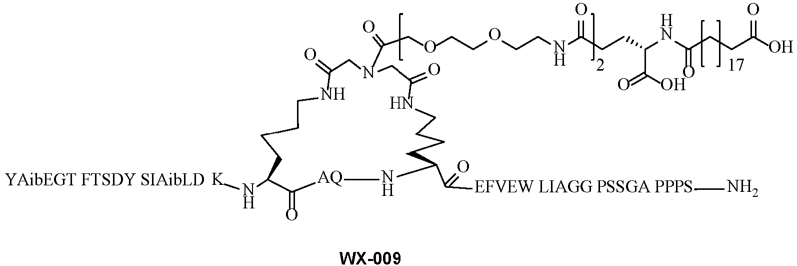

**Step 1:** 1.34 g of 4-(2', 4'-dimethoxyphenyl-fluorenemethoxycarbonyl-aminomethyl)-phenoxyacetamido-methylbenzhydrylamine resin (degree of substitution, Sub = 0.3 mmol/g) was weighed and added into a reaction column, into which was further added DMF (50 mL), and the reaction column was aerated with nitrogen for 2 h. After then, the waste was drained until no liquid flew out. The reaction column was washed with DMF (50 mL) for 5 times, each time for 1 min, and the waste was drained until no liquid flew out.
**Step 2:** 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.

### Step 3: Coupling of amino acids

### 3.1 Coupling of Fmoc-Ser(tBu)-OH

1. Fmoc-Ser(tBu)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.2 Coupling of Fmoc-Pro-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.
5. **Step 3.2 was similarly repeated to perform the coupling of the following amino acids.**

| **No.** | **Raw materials** | **Coupling reagents** |
|---|---|---|
| 3.3 | Fmoc-Pro-OH (3.00 eq) | HBTU (2.85 eq) and DIEA (6.00 eq) |
| 3.4 | Fmoc-Pro-OH (3.00 eq) | HBTU (2.85 eq) and DIEA (6.00 eq) |
| 3.5 | Fmoc-Ala-OH (3.00 eq) | HBTU (2.85 eq) and DIEA (6.00 eq) |
| 3.6 | Fmoc-Gly-OH (3.00 eq) | HBTU (2.85 eq) and DIEA (6.00 eq) |
| 3.7 | Fmoc-Ser(tBu)-OH (3.00 eq) | HBTU (2.85 eq) and DIEA (6.00 eq) |
| 3.8 | Fmoc-Ser(tBu)-OH (3.00 eq) | HBTU (2.85 eq) and DIEA (6.00 eq) |
| 3.9 | Fmoc-Pro-OH (3.00 eq) | HBTU (2.85 eq) and DIEA (6.00 eq) |
| 3.10 | Fmoc-Gly-OH (3.00 eq) | HBTU (2.85 eq) and DIEA (6.00 eq) |
| 3.11 | Fmoc-Gly-OH (3.00 eq) | HBTU (2.85 eq) and DIEA (6.00 eq) |
| 3.12 | Fmoc-Ala-OH (3.00 eq) | HBTU (2.85 eq) and DIEA (6.00 eq) |
| 3.13 | Fmoc-Ile-OH (3.00 eq) | HBTU (2.85 eq) and DIEA (6.00 eq) |
| 3.14 | Fmoc-Leu-OH (3.00 eq) | HBTU (2.85 eq) and DIEA (6.00 eq) |
| 3.15 | Fmoc-Trp(Boc)-OH (3.00 eq) | HBTU (2.85 eq) and DIEA (6.00 eq) |
| 3.16 | Fmoc-Glu(tBu)-OH (2.00 eq) | HBTU (1.90 eq) and DIEA (4.00 eq) |
| 3.17 | Fmoc-Val-OH (3.00 eq) | HBTU (2.85 eq) and DIEA (6.00 eq) |
| 3.18 | Fmoc-Phe-OH (3.00 eq) | HBTU (2.85 eq) and DIEA (6.00 eq) |
| 3.19 | Fmoc-Glu(tBu)-OH (6.00 eq) | HBTU (2.85 eq) and DIEA (6.00 eq) |
| 3.20 | Fmoc-Lys(Dde)-OH(3.00 eq) | HOBT (2.00 eq) and DIC (2.00 eq) |
| 3.21 | Fmoc-Gln(Trt)-OH (3.00 eq) | HBTU (2.85 eq) and DIEA (6.00 eq) |
| 3.22 | Fmoc-Ala-OH (3.00 eq) | HBTU (2.85 eq) and DIEA (6.00 eq) |
| 3.23 | Fmoc-Lys(Alloc)-OH (2.00 eq) | HBTU (2.85 eq) and DIEA (6.00 eq) |
| 3.24 | Fmoc-Lys(Boc)-OH (3.00 eq) | HBTU (2.85 eq) and DIEA (6.00 eq) |
| 3.25 | Fmoc-Asp(tBu)-OH (6.00 eq) | HATU (5.70 eq) and DIEA (12.0 eq) |
| 3.26 | Fmoc-Leu-OH (6.00 eq) | HATU (5.70 eq) and DIEA (12.0 eq) |
| 3.27 | Fmoc-Aib-OH (6.00 eq) | HATU (5.70 eq) and DIEA (12.0 eq) |
| 3.28 | Fmoc-Ile-OH (6.00 eq) | HOAT (6.00 eq) and DIC (6.00 eq) |
| 3.29 | Fmoc-Ser(tBu)-OH (6.00 eq) | HATU (5.70 eq) and DIEA (12.0 eq) |
| 3.30 | Fmoc-Tyr(tBu)-OH (6.00 eq) | HATU (5.70 eq) and DIEA (12.0 eq) |
| 3.31 | Fmoc-Asp(tBu)-OH (6.00 eq) | HATU (5.70 eq) and DIEA (12.0 eq) |
| 3.32 | Fmoc-Ser(tBu)-OH (6.00 eq) | HATU (5.70 eq) and DIEA (12.0 eq) |
| 3.33 | Fmoc-Thr(tBu)-OH (6.00 eq) | HATU (5.70 eq) and DIEA (12.0 eq) |
| 3.34 | Fmoc-Phe-OH (6.00 eq) | HATU (5.70 eq) and DIEA (12.0 eq) |
| 3.35 | Fmoc-Thr(tBu)-OH (6.00 eq) | HATU (5.70 eq) and DIEA (12.0 eq) |
| 3.36 | Fmoc-Gly-OH (6.00 eq) | HATU (5.70 eq) and DIEA (12.0 eq) |
| 3.37 | Fmoc-Glu(tBu)-OH (6.00 eq) | HOBT (6.00 eq) and DIC (6.00 eq) |
| 3.38 | Fmoc-Aib-OH (3.00 eq) | HATU (2.85 eq) and DIEA (6.00 eq) |
| 3.39 | Boc-Tyr(tBu)-OH (3.00 eq) | HATU (2.85 eq) and DIEA (6.00 eq) |

### 3.40 Removal of Alloc

1. PhSiH₃ (10.0 eq) and DCM (10 mL) were added into the reaction column, which was then aerated with nitrogen and added with Pd(PPh₃)₄ (0.1 eq), then aerated with nitrogen for 20 min. The reaction was carried out twice, and the waste was drained until no liquid flew out.
2. The reaction column was washed with DMF for 5 times (50 mL each time), each time for 1 min, and the waste was drained until no liquid flew out.

### 3.41 Coupling of Fmoc-Ida-OH

1. Fmoc-Ida-OH (4.0 eq) was weighed and added into the resin, DIEA (8.00 eq) plus 10 mL of DMF were supplemented into the reaction column, which was then aerated with nitrogen and added with HBTU (3.80 eq) after the amino acids were dissolved. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.42 Coupling of Intermediate A-1

1. 10% DBU in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. **Intermediate A-1** (1.50 eq) was weighed and added into the resin, DIEA (3.00 eq) plus 10 mL of DMF were supplemented into the reaction column, which was then aerated with nitrogen and added with HBTU (1.45 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.43 Removal of Dde

1. 3% hydrazine in DMF (50 mL) was added into the reaction column which was then aerated with nitrogen for 15 min, and the waste was drained. The reaction column was washed with DMF (50 mL) for 5 times, each time for 1 min, and the waste was drained until no liquid flew out. Ninhydrin was used for detection, with the resin appearing blue.

### 3.44 Amide ring closing

1. DIEA (3.0 eq) in DMF was added into the resin, then a solution of HATU (1.5 eq) in DMF was dropwise added into the reaction column slowly, which was then aerated with nitrogen. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.
4. The resin was shrunk with MeOH (50 mL) for 3 min each time. The waste was drained until no liquid flew out. The resin was poured out and dried for further use.

### Step 4: Cutting and crude peptide drying

4.1 A cleaving solution was formulated following the volumes below:

| **Reagents** | **Proportion** |
|---|---|
| **Trifluoroacetic acid** | 92.5 |
| **Triisopropylsilane** | 2.5 |
| **H₂O** | 2.5 |
| **3-mercaptopropionic acid** | 2.5 |

4.2 The peptide resin with the peptide was added into the formulated cleaving solution, and the resulted solution was shaken on a shaker for 2.5 h, and filtered. The filtrate was added into 10 times the volume of ice-cold isopropyl ether, the resulted mixture was centrifuged, and the centrifuged deposit was washed for 5 times with isopropyl ether. The washed product was then dried in vacuum for 2 h to get a crude peptide, which was purified to obtain a polypeptide **WX-009.** The molecular weight of the polypeptide was confirmed by ESI-MS, with a calculated value of [M+4H]/4 as 1247.1 and a detected value of 1247.2.

### Embodiment 10

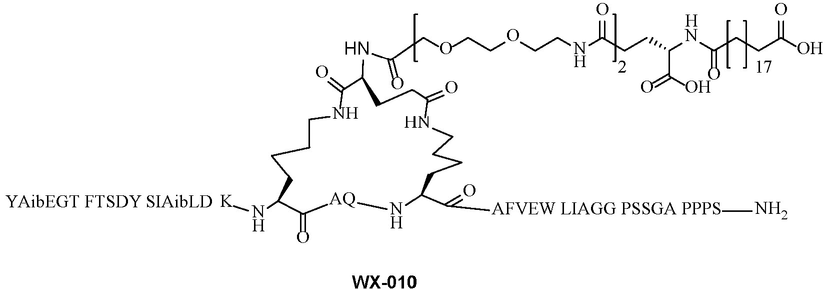

**Step 1:** 1.08 g of 4-(2', 4'-dimethoxyphenyl-fluorenemethoxycarbonyl-aminomethyl)-phenoxyacetamido-methylbenzhydrylamine resin (degree of substitution, Sub = 0.37 mmol/g) was weighed and added into a reaction column, into which was further added DMF (50 mL), and the reaction column was aerated with nitrogen for 2 h. After then, the waste was drained until no liquid flew out. The reaction column was washed with DMF (50 mL) for 5 times, each time for 1 min, and the waste was drained until no liquid flew out.
**Step 2:** 20% piperidine in DMF (50 mL) was added into the reaction column which was then aerated with nitrogen for 20 min. The waste was drained until no liquid flew out. The reaction column was washed with DMF (50 mL) for 5 times, each time for 1 min and the waste was drained until no liquid flew out. Ninhydrin was used for detection, with the resin appearing blue.

### Step 3: Coupling of amino acids

### 3.1 Coupling of Fmoc-Ser(tBu)-OH

1. Fmoc-Ser(tBu)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.2 Coupling of Fmoc-Pro-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.3 Coupling of Fmoc-Pro-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Pro-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.4 Coupling of Fmoc-Pro-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Pro-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.5 Coupling of Fmoc-Ala-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Ala-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.6 Coupling of Fmoc-Gly-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gly-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.7 Coupling of Fmoc-Ser(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.8 Coupling of Fmoc-Ser(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.9 Coupling of Fmoc-Pro-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.10 Coupling of Fmoc-Gly-Gly-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Gly-Gly-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.11 Coupling of Fmoc-Ala-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.12 Coupling of Fmoc-Ile-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ile-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.13 Coupling of Fmoc-Leu-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Leu-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.14 Coupling of Fmoc-Trp(Boc)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Trp(Boc)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.15 Coupling of Fmoc-Glu(OtBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Glu(OtBu)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.16 Coupling of Fmoc-Val-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Val-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.17 Coupling of Fmoc-Phe-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Phe-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.18 Coupling of Fmoc-Ala-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.19 Coupling of Fmoc-Lys(Alloc)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Alloc)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.20 Coupling of Fmoc-Gln(Trt)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gln(Trt)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.21 Coupling of Fmoc-Ala-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.22 Coupling of Fmoc-Lys(Dde)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Dde)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.23 Coupling of Fmoc-Lys(Boc)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Boc)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.24 Coupling of Fmoc-Asp(OtBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Asp(OtBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.25 Coupling of Fmoc-Leu-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Leu-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.26 Coupling of Fmoc-Aib-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min and the waste was drained until no liquid flew out. Ninhydrin was used for detection, with the resin appearing blue.
2. Fmoc-Aib-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 2 h. Ninhydrin was used for detection, with the resin appearing blue.
4. The reaction solution was drawn off from the reaction column which was then washed with DMF for 5 times (50 mL each time), each time for 1 min, and the waste was drained until no liquid flew out.

### 3.27 Coupling of Fmoc-Ile-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ile-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 1 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.28 Coupling of Fmoc-Ser(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.29 Coupling of Fmoc-Tyr(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Tyr(tBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.30 Coupling of Fmoc-Asp(OtBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Asp(OtBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.31 Coupling of Fmoc-Thr(tBu)-SerPsi(Me, Me)Pro-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Thr(tBu)-SerPsi(Me, Me)Pro-OH (2.00 eq) was weighed and added into the resin, DIEA (4.00 eq) plus 10 mL of DMF were supplemented into the reaction column, which was then aerated with nitrogen and added with HATU (1.90 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.32 Coupling of Fmoc-Phe-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Phe-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.33 Coupling of Fmoc-Thr(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Thr(tBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.34 Coupling of Fmoc-Gly-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gly-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.35 Coupling of Fmoc-Glu(OtBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Glu(OtBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.36 Coupling of Fmoc-Aib-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Aib-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C overnight. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.37 Coupling of Boc-Tyr(tBu)-OH

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Boc-Tyr(tBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was conducted at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.38 Removal of Alloc

1. PhSiH₃ (10.0 eq) and DCM (20 mL) were added into the reaction column, which was then aerated with nitrogen and added with Pd(PPh₃)₄ (0.10 eq), then aerated with nitrogen for 20 min. The reaction was carried out twice, and the waste was drained until no liquid flew out.
2. The reaction column was washed with DMF for 5 times (50 mL each time), each time for 1 min, and the waste was drained until no liquid flew out.

### 3.39 Coupling of Fmoc-Glu-OAll

1. Fmoc-Glu-OAll (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.40 Coupling of Intermediate A-1

1. 20% piperidine in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. **Intermediate A-1** (1.50 eq) was weighed and added into the resin, DIEA (3.00 eq) plus 10 mL of DMF were supplemented into the reaction column, which was then aerated with nitrogen and added with HATU (1.45 eq) after the amino acids were dissolved. Nitrogen was adjusted to make the resin bulge evenly.
3. The reaction was conducted at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.41 Removal of Alloc

1. PhSiH₃ (10.0 eq) and DCM (20 mL) were added into the reaction column, which was then aerated with nitrogen and added with Pd(PPh₃)₄ (0.10 eq), and then aerated with nitrogen for 20 min. The reaction was carried out twice, and the waste was drained until no liquid flew out.
2. The reaction column was washed with DMF for 5 times (50 mL each time), each time for 1 min, and the waste was drained until no liquid flew out.

### 3.42 Removal of Dde

1. 3% hydrazine hydrate in DMF (50 mL) was added into the reaction column which was then aerated with nitrogen for 15 min, and the waste was drained. The reaction column was washed with DMF (50 mL) for 5 times, each time for 1 min, and the waste was drained until no liquid flew out. Ninhydrin was used for detection, with the resin appearing blue.

### 3.43 Amide ring closing

1. DIEA (3.00 eq) in DMF was added into the resin, then a solution of HATU (1.50 eq) in DMF was dropwise added into the reaction column slowly, which was then aerated with nitrogen. Nitrogen was adjusted to make the resin bulge evenly.
2. The reaction was conducted at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.
4. The resin was shrunk with MeOH (50 mL) for 3 min each time. The waste was drained until no liquid flew out. The resin was poured out and dried for further use.

### Step 4: Cutting and crude peptide drying

4.1 A cleaving solution was formulated following the volumes below:

| **Reagents** | **Proportion** |
|---|---|
| **Trifluoroacetic acid** | 92.5 |
| **Triisopropylsilane** | 2.5 |
| **H₂O** | 2.5 |
| **3-mercaptopropionic acid** | 2.5 |

4.2 The dried resin with the peptide was added into the formulated cleaving solution, and the resulted solution was shaken on a shaker for 2.5 h, and filtered. The filtrate was added into 10 times the volume of ice-cold isopropyl ether, the resulted mixture was centrifuged, and the centrifuged deposit was washed for 5 times with isopropyl ether. The washed product was then dried in vacuum for 2 h to get a crude peptide, which was purified. The molecular weight of the polypeptide was confirmed by ESI-MS, with a calculated value of 1236.1 and a detected value of 1236.0.

### Embodiment 11

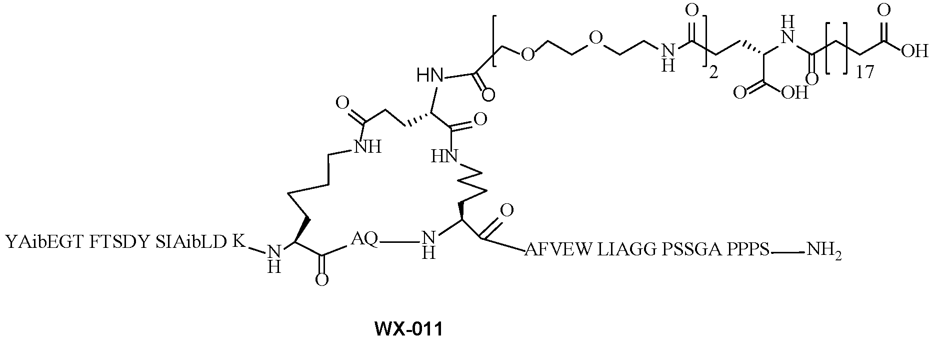

With reference to the synthesis of WX-010, a polypeptide WX-011 was obtained. The molecular weight of the polypeptide was confirmed by ESI-MS, with a calculated value of [M+4H]/4 as 1236.1 and a detected value of 1236.0.

### Embodiment 12

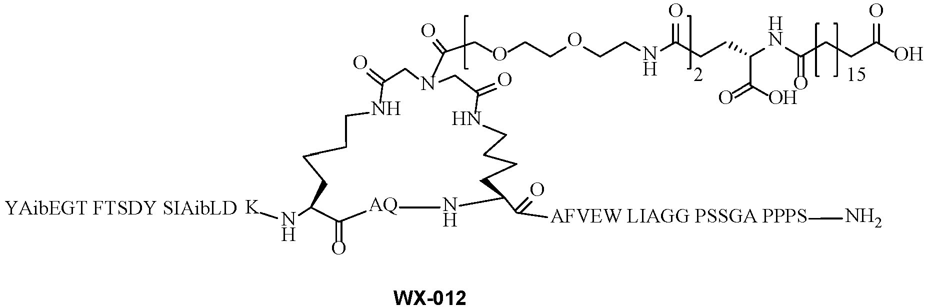

With reference to the synthesis of **WX-001** and using the intermediate **A-2,** a polypeptide **WX-012** was obtained. The molecular weight of the polypeptide was confirmed by ESI-MS, with a calculated value of [M+4H]/4 as 1225.6 and a detected value of 1225.7.

### Embodiment 13

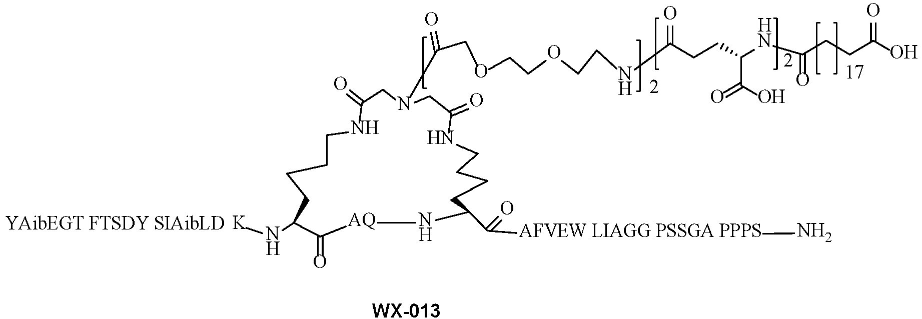

With reference to the synthesis of **WX-001** and using the intermediate **A-3,** a polypeptide **WX-**013 was obtained. The molecular weight of the polypeptide was confirmed by ESI-MS, with a calculated value of [M+4H]/4 as 1264.9 and a detected value of 1264.6.

### Embodiment 14

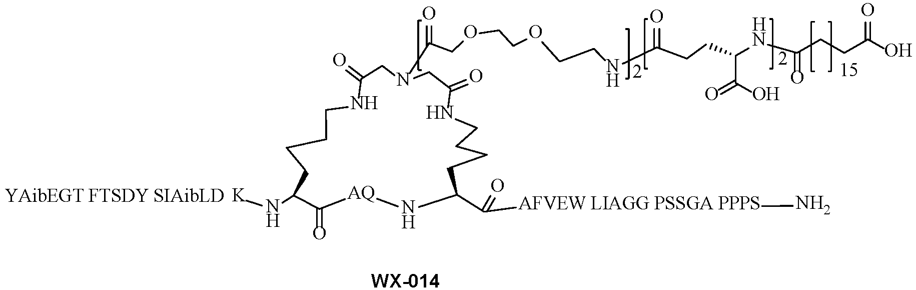

With reference to the synthesis of **WX-001** and using the intermediate **A-4,** a polypeptide **WX-**014 was obtained. The molecular weight of the polypeptide was confirmed by ESI-MS, with a calculated value of [M+4H]/4 as 1257.9 and a detected value of 1257.8.

### Biological test data

### Test example 1: In vitro GLP-1R/GIPR agonistic activity test

### A. Main Materials:

### 1) Cell lines

The cell lines were constructed by Wuxi APPTEC (Shanghai) Co., Ltd. See details in Table 1 below.

**Table 1. Cell Lines Information**

| Target | Host cell | Clone |
|---|---|---|
| GLP-1R | HEK293 | N/A |
| GIPR | CHO | N/A |

### 2) Reagents and Consumables

**Table 2. Reagents and Consumables Information**

| Name | Lot No. | Item No. | Manufacturer |
|---|---|---|---|
| cAMP kit | 29F | 62AM4PEJ | Cisbio |
| 1M HEPES | 2120919 | 15630-106 | Invitrogen |
| Hanks' Balanced Salt Solution (HBSS) | 2185775 | 14025 | Invitrogen |
| Human serum albumin (HSA) | SLCF7301 | A1653-10G | Sigma |
| Casein | SLCC9458 | C4765-10 mL | Sigma |
| 3-isobutyl-1-methylxanthine (IBMX) | STBF6061V | I5879-5G | Sigma |
| ECHO qualified 384-well plate | 0006433672 | PP-0200 | Labcyte |
| OptiPlate-384 | 8210-19481 | 6007299 | PerkinElmer |

### 3) Instruments

**Table 3. Instruments Information**

| Name | Model | Manufacturer |
|---|---|---|
| EnVision | envision2014 | PerkinElmer |
| Vi-cell counter | Vi-CELL^{™} XR Cell Viability Analyzer | Beckman |
| Bravo | Bravo V11 | Agilent |
| ECHO | ECHO 555 | Labcyte |
| Centrifuge | Allegra^{™} 25R Centrifuge | Beckman |

### B. Methods

### 1) Experimental Materials

### Experimental buffers

**Table 4. Buffers Information**

| **Reagents** | **Stock concentration** | **Volume** | **Final concentration** |
|---|---|---|---|
| Hanks' Balanced Salt Solution | 1× | 48.7 mL/44.7 mL | ≈1x |
| HEPES buffer | 1 mol/L | 250 µL | 5 mmol/L |
| 5% casein solution (HEPES)/ 10% human serum albumin solution (HSA) | 5%/ 10% | 1000 µL/ 5000 µL | 0.10%/ 1% |
| 3-isobutyl-1-methylxanthine (IBMX) | 500 mmol/L | 50 µL | 0.5 mmol/L |

### Preparation of assay reagents

**Table 5. Assay Reagents Information**

| **Reagents** | **Stock concentration** | **Volume** | **Final concentration** |
|---|---|---|---|
| Cell lysis buffer | 1× | 9.5 mL | ≈1x |
| D2-cAMP solution | 40× | 250 µL | 1× |
| cAMP-antibody solution | 40× | 250 µL | 1× |

### 2) Experimental Methods

### a) Preparation of compound plates:

The test compounds were diluted at a 4-fold dilution factor in 10 points, starting from an initial concentration of 30 µM. The dilution was conducted by Bravo.

### b) Transfer of compounds:

1) 100 nL of compounds were transferred to an OptiPlate-384 plate by Echo.
2) The OptiPlate-384 plate was centrifuged at 1000 rpm for 5 s.

### c) Preparation of cell suspension:

1) A GLP-1R/GIPR cell cryovial was placed in warm water at 37°C for rapid thawing.
2) The cell suspension was transferred into a 15 mL Transfer centrifugal tube and rinsed with 10 mL of HBSS gently.
3) The centrifugal tube was centrifuged at 1000 rpm at room temperature for 1 min.
4) The supernatant was discarded.
5) The bottom cells were gently dispersed, then gently rinsed with 10 mL of HBSS, the centrifugal tube was centrifuged to settle the cells, and finally the cells were resuspended in an experimental buffer.
6) Cell density and viability were measured by Vi-cell.
7) The GLP-1R/GIPR cells were diluted with the experimental buffer to a concentration of 2.0*10⁵/mL.
8) 100 nL of the diluted cell suspension was transferred into the OptiPlate-384 plate.
9) Incubation was performed at room temperature for 30 min.

### d) An assay reagent was added:

1) 10 µL of 800 nM gradient diluted cAMP standard was added into the empty wells of the OptiPlate-384 plate.
2) 10 µL of cAMP assay reagent was added.
3) The OptiPlate-384 plate was covered with Top Seal -A film and incubated at room temperature for 60 min.
4) The TopSeal-A film was removed, and readout was performed on EnVision.

### C. Experimental results

The experimental results were shown in Table 6 below.

**Table 6. In vitro GLP-1R/GIPR agonistic activity test results**

| **Polypeptide drugs** | **Agonistic activity on GLP-1R** | | | **Agonistic activity on GIPR** | | |
|---|---|---|---|---|---|---|
| | EC₅₀ (nM) | | Ratio | EC₅₀ (nM) | | Ratio |
| | (-) 1% HSA | (+) 1% HSA | | (-) 1% HSA | (+) 1% HSA | |
| **WX-001** | 0.05958 | 16.6 | 278.6 | 0.3239 | 15.97 | 49.3 |
| **WX-002** | 0.19 | 31.35 | 165 | 0.43 | 12.49 | 29 |
| **WX-003** | 0.54 | 13.97 | 26 | 1.07 | 43.95 | 41 |
| **WX-004** | 1.86 | 84.28 | 45 | 1.36 | 21.37 | 16 |
| **WX-005** | 0.036 | 7.63 | 212 | 0.16 | 19.29 | 121 |
| **WX-006** | 0.027 | NA | NA | 0.26 | NA | NA |
| **WX-007** | 0.026 | NA | NA | 0.40 | NA | NA |
| **WX-008** | 0.18 | NA | NA | 2.74 | NA | NA |
| **WX-009** | 0.014 | NA | NA | 0.074 | NA | NA |
| **WX-010** | 0.017 | NA | NA | 0.14 | NA | NA |
| **WX-011** | 0.018 | NA | NA | 0.25 | NA | NA |
| **WX-012** | 0.024 | NA | NA | 0.17 | NA | NA |
| **WX-013** | 0.057 | NA | NA | 0.18 | NA | NA |
| **WX-014** | 0.071 | NA | NA | 0.24 | NA | NA |

| | | | | | | |
|---|---|---|---|---|---|---|
| **Conclusion:** The compounds of the present application exhibited strong agonistic activity towards GLP-1R/GIPR. | | | | | | |

### Test example 2: Evaluation of pharmacokinetic profiles of the compounds in rats

### A. Experimental purpose:

To test the pharmacokinetic profiles of the compounds in SD rats.

### B. Experimental procedure

The pharmacokinetic characteristics in rodent animals following subcutaneous injection of the compounds were tested using standard protocols. In the experiment, the candidate compounds were formulated into clear solution, which was administered to the rats by single subcutaneous injection (SC, 0.048 mpk). The vehicle for injection was citrate buffer solution (20 mM, pH=7). The whole blood was collected and processed to get the plasma. The drug concentration was analyzed by LC-MS/MS, and the pharmacokinetic parameters were calculated using Phoenix WinNonlin software.

### C. Experimental results

The experimental results were shown in Table 7 below.

**Table 7. Pharmacokinetic test results**

| **Compound No.** | **Peak concentration of drugs (nM)** | **Half-life T_{1/2} (h)** | **Peak time Tₘₐₓ (h)** | **SC concentration integration AUC₀₋₇₂ₕ (nM.hr)** |
|---|---|---|---|---|
| **WX005** | 28 | 21 | 16 | 1153 |
| **WX009** | 24 | 21 | 20 | 1092 |

| | | | | |
|---|---|---|---|---|
| **Conclusion:** The compounds of the present application exhibited excellent pharmacokinetic properties in rats. | | | | |

### Test example 3: Evaluation of pharmacokinetic profiles of the compounds in mice

### A. Experimental purpose:

To test the pharmacokinetic profiles of the compounds in C57BL/6 mice.

### B. Experimental procedure

The pharmacokinetic characteristics in rodent animals following subcutaneous injection of the compounds were tested using standard protocols. In the experiment, the candidate compounds were formulated into clear solution, which was administered by subcutaneous injection (SC, 0.048 mpk). The vehicle for subcutaneous injection was citrate buffer solution (20 mM, pH=7). The whole blood was collected and processed to get the plasma. The drug concentration was analyzed by LC-MS/MS, and the pharmacokinetic parameters were calculated using Phoenix WinNonlin software.

### C. Experimental results

The experimental results were shown in Table 8 below.

**Table 8. Pharmacokinetic test results**

| **Compound No.** | **Peak concentration of drugs (nM)** | **Half-life T_{1/2} (h)** | **Peak time Tₘₐₓ (h)** | **SC concentration integration AUC₀₋₇₂ₕ (nM.hr)** |
|---|---|---|---|---|
| **WX005** | 79 | 16 | 12 | 2240 |
| **WX009** | 59 | 17 | 12 | 1986 |

| | | | | |
|---|---|---|---|---|
| **Conclusion:** The compounds of the present application exhibited excellent pharmacokinetic properties in mice. | | | | |

### Test example 4: Evaluation of pharmacokinetic profiles of the compounds in Cynomolgus macaques

### A. Experimental purpose:

To test the pharmacokinetic profiles of the compounds in Cynomolgus macaques.

### B. Experimental procedure

The pharmacokinetic characteristics in mammals following intravenous injection and subcutaneous injection of the compounds were tested using standard protocols. In the experiment, the candidate compounds were formulated into clear solution, which was administered to Cynomolgus macaques by single subcutaneous injection (SC, 0.02 mpk). The vehicle for subcutaneous injection was citrate buffer solution (20 mM, pH=7). The whole blood was collected and processed to get the plasma. The drug concentration was analyzed by LC-MS/MS, and the pharmacokinetic parameters were calculated using Phoenix WinNonlin software.

### C. Experimental results

The experimental results were shown in Table 9 below.

**Table 9. Pharmacokinetic test results**

| **Compound No.** | **Peak concentration of drugs (nM)** | **Half-life T_{1/2} (h)** | **Peak time Tₘₐₓ (h)** | **SC concentration integration AUC₀₋₇₂ₕ (nM.hr)** |
|---|---|---|---|---|
| **WX005** | 36 | 100 | 24 | 4467 |
| **WX009** | 37 | 105 | 24 | 4737 |

| | | | | |
|---|---|---|---|---|
| **Conclusion:** The compounds of the present application exhibited excellent pharmacokinetic properties in monkeys. | | | | |

### Test example 5: Plasma stability test (PLS)

### A. Experimental purpose:

To study the stability of the test compounds in the plasma of normal mice.

### B. Experimental procedure

1. Before the experiment, the clotted frozen plasma was thawed in a water bath at 37°C. The resulting plasma was centrifuged at 4000 rpm for 5 min. If there is blood clot, remove the clot. The pH was adjusted to 7.4±0.1.
2. The test compound solution was prepared by diluting the compounds with DMSO to prepare a solution of 100 µM.
3. 98 µL of plasma, as a blank control, was added with 2 µL of the test compound solution (100 µM) to get a mixed solution with a final concentration of 2 µM, which was incubated in a water bath at 37°C.
4. 100 µL of H₃PO₄ solution and 800 µL of stop solution (a solution of 200 ng/mL tolbutamide and 200 ng/mL labetalol in 100% methanol) were respectively added at each time point (0, 10, 30, 60, and 120 min) to precipitate protein, which was then mixed thoroughly.
5. The samples were centrifuged at 4000 rpm for 20 min, and 100 µL of supernatant was taken from each well for LC-MS/MS analysis.

### C. Experimental results

The experimental results were shown in Table 10 below.

**Table 10. PLS test results**

| **Compound No.** | **WX005** | **WX009** |
|---|---|---|
| PLS (H/M) T_{1/2} (min) | >289.1/>289.1 | >289.1/>238.9 |

| | | |
|---|---|---|
| **Conclusion:** The compounds of the present application exhibited excellent plasma stability. | | |

### Test example 6: Plasma protein binding test (PPB)

### A. Experimental purpose:

To study the binding of the test compounds to human/mouse plasma albumin.

### B. Experimental procedure

1. Blank matrix preparation: On the day of the experiment, the plasma was thawed in cold water and centrifuged at 3220 rpm for 5 min to remove all the blood clots. The pH of the resulting plasma was measured and adjusted to 7.4±0.1 with 1% phosphoric acid or 1 N sodium hydroxide as desired.
2. Dilution of the test compounds: the test compounds were dissolved in dimethyl sulfoxide (DMSO) to prepare stock solutions of 10 mM and 2 mM, respectively. 2 µL of the stock solution (2 mM) was diluted with 98 µL of DMSO to obtain a working solution of 40 µM. 10 µL of the stock solution was diluted with 240 µL of DMSO to obtain a working solution of control compounds of 400 µM. A working solution of the compounds (5 µL) was mixed evenly with a blank matrix (995 µL) at a ratio of 1:200 to prepare a loading matrix.
3. Analytical procedure

3.1 Equal amounts of 30 µL of the loading matrix (n=2) were transferred into a sample collection plate to prepare Time 0 (T0) samples for residue determination. The samples were immediately matched with corresponding blank buffer to a final volume of 60 µL, with a volume ratio of plasma (the loading matrix) to buffer being 1: 1 in each well. Then, into the T0 samples of the test compounds were respectively added 60 µL of 4% H₃PO₄ in H₂O and 480 µL of stop solution containing internal standard. They were then stored at 2°C to 8°C together with other samples for further treatment.

3.2 The remaining plasma samples were pre-incubated in a carbon dioxide incubator at 37±1°C for 30 min. Protein-free samples (F samples) were prepared. Matrix-loaded samples (230 µL) were all transferred into polycarbonate tubes (n = 2) and ultracentrifuged at 37°C and 155,000 × g (35000 rpm) for 4 h.

3.3 To prepare T samples (test samples), an additional matrix-containing sample was transferred into a separate 96-well plate (sample incubation plate) and incubated at 37°C for 4 h.

3.4 At the end of centrifugation, 30 µL of protein-free samples (F samples) and 30 µL of T samples were taken from the second layer (beneath the upper layer) of the supernatant and transferred into a new sample collection plate. Each sample was mixed with corresponding blank buffer or matrix to a final volume of 60 µL, and the blank matrix : buffer volume ratio is 1:1. Into all the samples were added 60 µL of 4% H₃PO₄ solution in water and 480 µL of stop solution containing internal standard. The mixture was centrifuged at 4000 rpm for 20 min. 100 µL of supernatant for each sample was taken for LC-MS/MS analysis.

### C. Experimental results

The experimental results were shown in Table 11 below.

**Table 11. PPB test results**

| **Compound No.** | **WX005** | **WX009** |
|---|---|---|
| PPB% unbound (HIM) | NA/NA | NA/NA |

| | | |
|---|---|---|
| Note: NA indicates that the plasma protein binding was too high and no free drugs were detected at a normal plasma protein concentration. **Conclusion:** The compounds of the present application exhibited extremely high plasma protein binding. | | |

### Test example 7: In vivo efficacy test in mouse model of obesity

### A. Experimental purpose:

To study the weight-lowering effect of the series of polypeptides of the present application in animal model of obesity, and to compare them with the marketed counterparts, Tirzepatide and Semaglutide.

### B. Experimental procedure

### 1. Establishment of animal model

Mouse model of obesity was obtained by induction with high-fat diet following the reference literature (MOLECULAR METABOLISM, 18 (2018), P3-14). The specific experimental procedure was as follows: the experimental mice were fed with high-fat diet (Research Diet D12492) for 13 weeks. The animals in both the control group and the model animals exhibited a steady increase in body weight. However, the animals in the model group showed significantly higher body weight compared to those in the control group, accompanied by markedly increased food intake. These findings indicated the successful establishment of animal models.

### 2. Dosing

The test animals were divided into groups. An appropriate amount of the test samples were added into 20 mM sodium citrate buffer following the experimental protocol, and the mixture was vortexed at room temperature to obtain clear solutions of varying concentrations. The obtained solutions were frozen for further use. During the experiment, animals in the model group and the dosing group were administered by means of subcutaneous injection on the outer thighs. The dosing regimen was once every 3 days (Q3d), for a total period of 22 days. Details were as follows.

| Groups | Dosage | Dosing frequency | Animal Number |
|---|---|---|---|
| G1: Normal group | / | / | 8 |
| G2: Model blank group | Blank solvent (20 mM sodium citrate buffer) | Q3d | 8 |
| G3: WX001 | 10 nmol/kg | Q3d | 8 |
| G4: WX005 | 10 nmol/kg | Q3d | 8 |
| G5: Tirzepatide | 10 nmol/kg | Q3d | 8 |
| G6: Semaglutide | 30 nmol/kg | Q3d | 8 |

### C. Results and analysis

Throughout the experimental procedure, all the test mice exhibited good health and mental status. The animals in the dosing group showed different degrees of inhibition of food intake under the action of the drug, but none of them died. After completing the entire dosing test cycle, the test mice were weighed, and the results were shown below.

| Groups | Weight change rate |
|---|---|
| G1: Normal group | -1.20% |
| G2: Model blank group | 8.60% |
| G3: WX001 | -9.35% |
| G4: WX005 | -17.00% |
| G5: Tirzepatide | -10.20% |
| G6: Semaglutide | -8.92% |

| | |
|---|---|
| **Conclusion:** Both WX001 and WX005 exhibited good weight-lowering effect in mouse model of obesity, with WX005 exhibiting better efficacy and being superior to Tirzepatide and Semaglutide. | |

In repeated experiments, the test drugs also exhibited substantially consistent effect in the corresponding groups, indicating the objective efficacy advantage.

Furthermore, the weight-lowering advantageous effect was also observed in other polypeptide compounds of the present application.

### Test example 8: In vivo efficacy test in mouse model of diabetes

### A. Experimental purpose:

To study the efficacy of the series of polypeptides of the present application in animal model of diabetes, and to compare them with the marketed counterpart Tirzepatide as reported in the literature (MOLECULAR METABOLISM, 18 (2018), P3-14). The glucose-lowering effect of Tirzepatide is much better than that of Semaglutide, and thus Semaglutide was not included in this experiment for comparison.

### B. Experimental procedure

### 1. Selection of animal model

Db/db (Lepr leptin receptor deficient) mice have typical clinical symptoms of diabetes such as extreme obesity, hyperphagia, thirst, and polyuria, and they are ideal animal model of type II diabetes.

The specific testing procedure was as follows: db/db test mice which were weighed greater than 30 g and had a fasting blood glucose level greater than 15 mmol/L were screened to be included in the formal trial.

### 2. Dosing:

The test animals were divided into groups. An appropriate amount of the test samples were added into 20 mM sodium citrate buffer following the experimental protocol, and the mixture was vortexed at room temperature to obtain clear solutions of varying concentrations. The obtained solutions were frozen for further use.

During the experiment, animals in the normal control group, the model group and the dosing group were administered by means of subcutaneous injection on the outer thighs. The dosing regimen was once a day (Qd), for a total period of 4 weeks. Details were as follows:

| Groups | Dosage | Dosing frequency | Animal Number |
|---|---|---|---|
| G1: Normal group | Saline | Qd | 8 |
| G2: Model blank group | Blank solvent (20 mM sodium citrate buffer) | Qd | 8 |
| G3: WX005 | 5 nmol/kg | Qd | 8 |
| G4: WX009 | 5 nmol/kg | Qd | 8 |
| G5: Tirzepatide | 5 nmol/kg | Qd | 8 |

### C. Results and analysis

Throughout the experimental procedure, all the test mice exhibited good health and mental status. The animals in the dosing group showed different degrees of inhibition of food intake under the action of the drug, but none of them died. The fasting blood glucose level and the change rate of glycosylated hemoglobin level in the test mice were dynamically monitored during the experiment. The results of the fasting blood glucose level were shown below.

| Groups | Fasting blood glucose (mmol/L) | | | |
|---|---|---|---|---|
| | Day 0 | Day 7 | Day 14 | Day 21 |
| G1: Normal group | 7.7±0.2 | 7.3±0.4 | 8.9±0.4 | 7.3±0.5 |
| G2: Model blank group | 26.8±1.3 | 24.1±1.6 | 29.9±1.7 | 28.3±0.8 |
| G3: WX005 | 26.4±1.5 | 11.7±1.2 | 13.8±2.0 | 12.8±1.8 |
| G4: WX009 | 26.4±1.2 | 11.8±1.8 | 12.6±2.2 | 13.6±1.7 |
| G5: Tirzepatide | 26.7±1.4 | 11.6±1.4 | 12.6±1.6 | 14.0±1.4 |

The results of the change rate of glycosylated hemoglobin level were shown below.

| Groups | Change rate of glycosylated hemoglobin (%) | | |
|---|---|---|---|
| | Day 14 | Day 21 | Day 29 |
| G1: Normal group | 4.8±2.1 | -5.0±4.4 | 5.5±5.6 |
| G2: Model blank group | -0.9±2.6 | 5.3±1.8 | 16.6±3.1 |
| G3: WX005 | -25.1±4.4 | -25.6±4.8 | -27.4±4.6 |
| G4: WX009 | -25.3±3.2 | -24.8±3.9 | -26.4±5.9 |
| G5: Tirzepatide | -15.9±5.4 | -14.3±4.9 | -10.2±5.7 |

| | | | |
|---|---|---|---|
| Note: "-" indicates decrease. **Conclusion:** Both WX005 and WX009 exhibited good glucose-lowering effect in mouse model of diabetes. Specifically, WX005 and WX009 exhibited similar effect in lowering the glycosylated hemoglobin level in the test animals, being superior to Tirzepatide. WX005 was slightly superior to WX009 in terms of lowering the fasting blood glucose level in the test animals, and both of them were superior to Tirzepatide. Overall, WX005 and WX009 were considered having significantly better glucose-lowering effect than Tirzepatide. | | | |

In repeated experiments, the test drugs also exhibited substantially consistent effect in the corresponding groups, indicating the objective efficacy advantage.

Furthermore, the efficacy advantages in terms of glucose metabolism in animals were also observed in other polypeptide compounds of the present application.

## Claims

1. A pharmaceutical use of a polypeptide having a sequence as shown in formula (II) in the preparation of a drug for treating and/or preventing diabetes, obesity, and related diseases thereof,
YAibEGT FTSDY SIAibLD KIAQK AFVKW LIAGG PSSGA PPPS₀ **(II)**
wherein the polypeptide has modifications as below:
1) the amino acid at position i is replaced by lysine and then the amino group on the lysine at position i is attached to 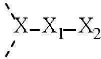 together with the amino group on the side chain of the lysine at position i+3, or the amino groups on the side chains of the lysine at positions j and j+4 are attached to 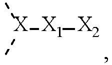 wherein i is 17, or wherein j is 20; and
2) additional 0 to 2 amino acids of the polypeptide of the sequence as shown in formula (II) are replaced;
wherein,
Aib has a structure of 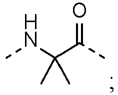
S₀ is selected from the group consisting of 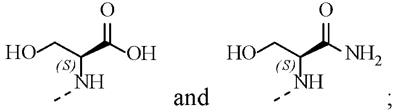
X is selected from the group consisting of 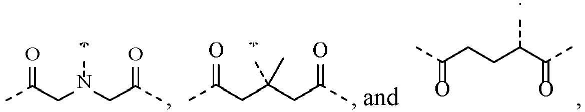
wherein "*" indicates the position attached to X₁;
X₁ is selected from the group consisting of a single bond, -C(=O)-, -O-C(=O)-, and -N(R₁)-C(=O)-;
Ri is selected from the group consisting of H and C₁₋₃ alkyl;
X₂ is selected from the group consisting of 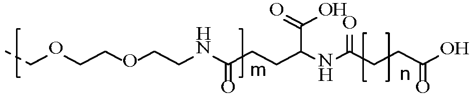 and 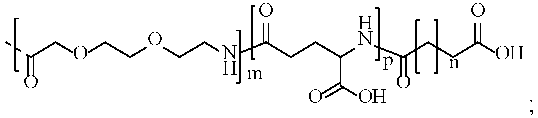
m is selected from 2, 3, and 4;
n is selected from 15, 16, 17, 18, and 19;
p is selected from 1 and 2.

2. The pharmaceutical use of the polypeptide according to claim 1, wherein the sequence of the polypeptide is as shown in formula **(P),**
YAibEGT FTSDY SIAibLD KKAQK AFVKW LIAGG PSSGA PPPS₀ **(P)**
which has modifications as below:
1) the amino groups on the side chains of the lysine at positions 17 and 20 are attached to 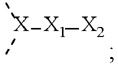 and
2) 0 to 2 amino acids of the polypeptide are replaced; wherein,
Aib has a structure of 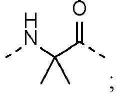
S₀ is selected from the group consisting of 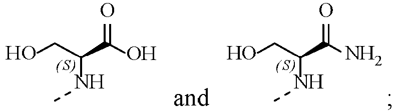
X is selected from the group consisting of 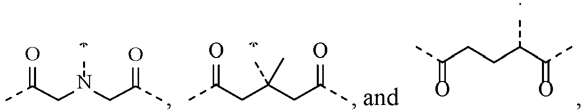
wherein "*" indicates the position attached to X₁;
X₁ is selected from the group consisting of a single bond, -C(=O)-, -O-C(=O)-, and -N(R₁)-C(=O)-;
Ri is selected from the group consisting of H and C₁₋₃ alkyl;
X₂ is selected from the group consisting of 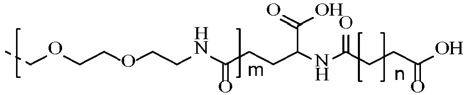 and 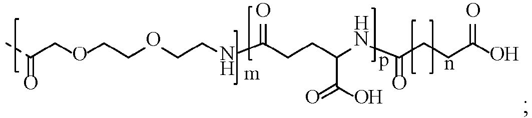
m is selected from 2, 3, and 4;
n is selected from 15, 16, 17, 18, and 19;
p is selected from 1 and 2.

3. The pharmaceutical use of the polypeptide according to claim 1 or 2, wherein, 0 to 2 amino acids at positions 21, 23 and 24 of the polypeptide are replaced.

4. The pharmaceutical use of the polypeptide according to claim 1, wherein the polypeptide has a sequence selected from the group consisting of sequences as shown in formulas (II-1), (II-2), (II-3), (II-4), (III-6), (IV-7), (IV-8), (IV-9), and (IV-10),
YAibEGT FTSDY SIAibLD KIAQK AFVKW LIAGG PSSGA PPPS-NH₂ **(II-1)**
YAibEGT FTSDY SIAibLD KIAQK EFVKW LIAGG PSSGA PPPS-NH₂ **(II-2)**
YAibEGT FTSDY SIAibLD KIAQK AFIKW LIAGG PSSGA PPPS-NH₂ **(II-3)**
YAibEGT FTSDY SIAibLD KIAQK AFVKW LLAGG PSSGA PPPS-NH₂ **(II-4)**
YAibEGT FTSDY SIAibLD KKAQK AFVEW LIAGG PSSGA PPPS-NH₂ **(III-6)**
YAibEGT FTSDY SIAibLD KKAQK AFVQW LIAGG PSSGA PPPS-NH₂ **(IV-7)**
YAibEGT FTSDY SIAibLD KKAQK AFVAW LIAGG PSSGA PPPS-NH₂ **(IV-8)**
YAibEGT FTSDY SIAibLD KKAQK AFVIW LIAGG PSSGA PPPS-NH₂ **(IV-9)**
YAibEGT FTSDY SIAibLD KKAQK EFVEW LIAGG PSSGA PPPS-NH₂ **(IV-10)**
which has modifications as below:
the amino groups on the side chains of the lysine at positions 17 and 20 or the amino groups on the side chains of the lysine at positions 20 and 24 are attached to 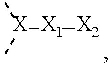 wherein, Aib, X, X₁, and X₂ are as defined in claim 1.

5. The pharmaceutical use of the polypeptide according to any one of claims 1 to 4, wherein, Ri in the polypeptide is selected from H.

6. The pharmaceutical use of the polypeptide according to any one of claims 1 to 4, wherein, X₁ in the polypeptide is selected from the group consisting of a single bond, -C(=O)-, -O-C(=O)-, and -NH-C(=O)-.

7. The pharmaceutical use of the polypeptide according to any one of claims 1 to 4, wherein, m in the polypeptide is selected from 2.

8. The pharmaceutical use of the polypeptide according to any one of claims 1 to 4, wherein, n in the polypeptide is selected from 15 and 17.

9. The pharmaceutical use of the polypeptide according to any one of claims 1 to 4, wherein, p in the polypeptide is selected from 2.

10. The pharmaceutical use of the polypeptide according to any one of claims 1 to 4, wherein, X₂ in the polypeptide is selected from the group consisting of 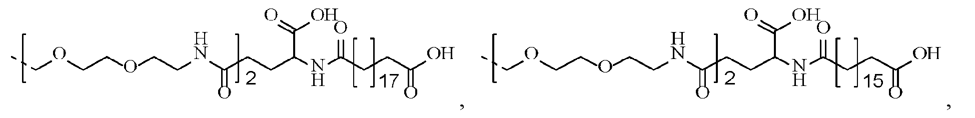 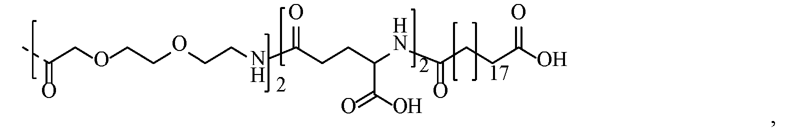 and

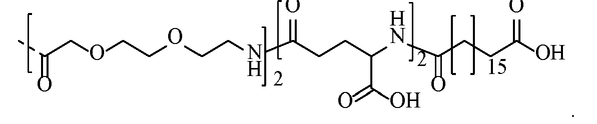

11. The polypeptide according to claim 10, wherein, X₂ is selected from the group consisting of 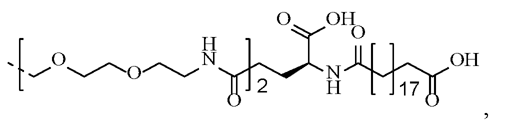 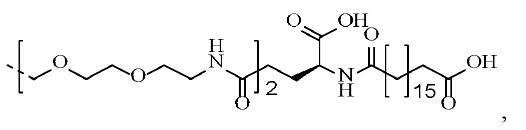 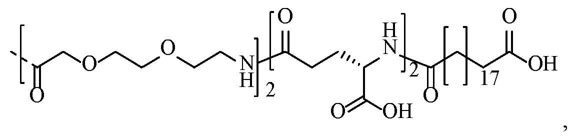 and

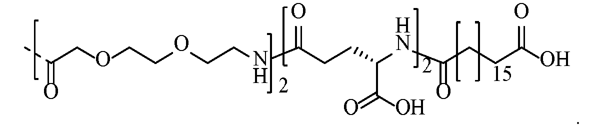

12. The polypeptide according to any one of claims 1 to 4, wherein, the amino groups on the side chains of the lysine at positions i and i+3 or the amino groups on the side chains of the lysine at positions j and j+4 are attached to 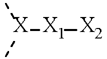 to form

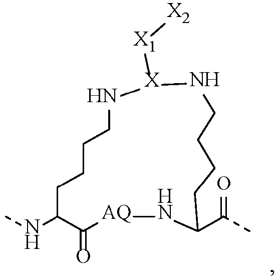

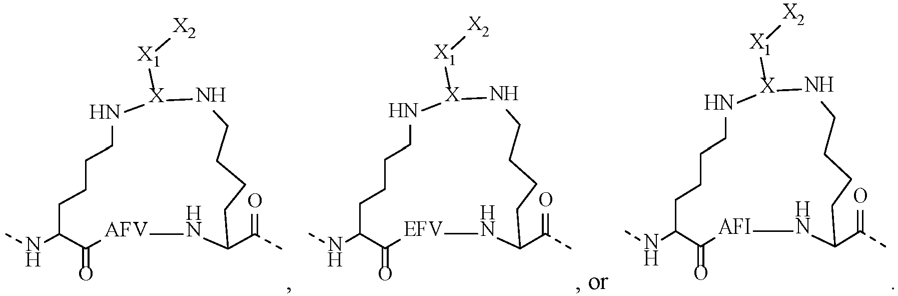

13. The polypeptide according to any one of claims 1 to 4, wherein, the amino groups on the side chains of the lysine at positions i and i+3 or the amino groups on the side chains of the lysine at positions j and j+4 are attached to 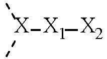 to form

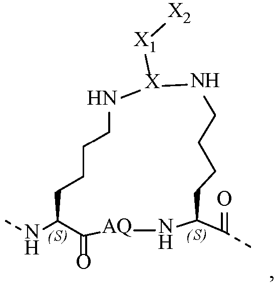

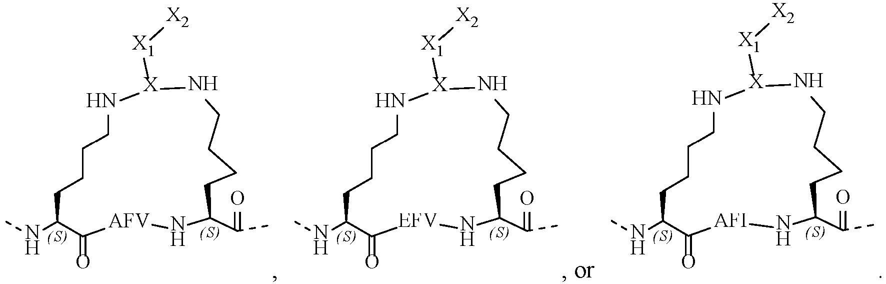

14. The pharmaceutical use of the polypeptide according to any one of claims 1 to 4, wherein, the structural unit 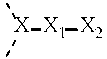 in the polypeptide is selected from the group consisting of 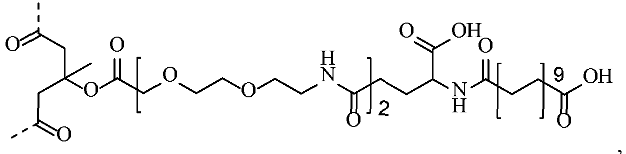 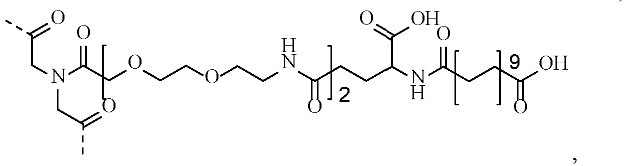 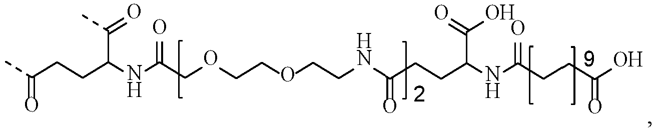 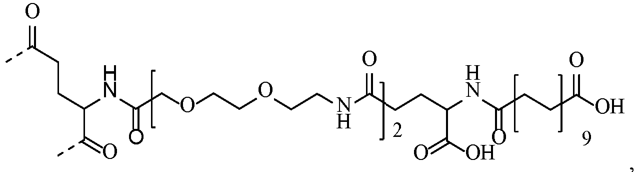 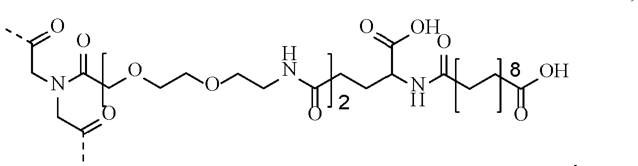 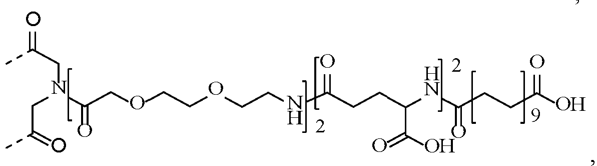 and

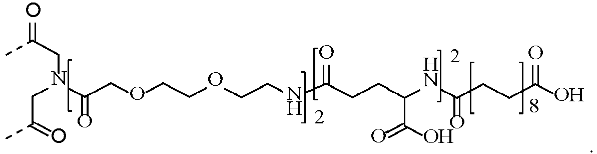

15. The pharmaceutical use of the polypeptide according to claim 14, wherein, the structural unit 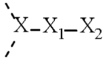 in the polypeptide is selected from the group consisting of 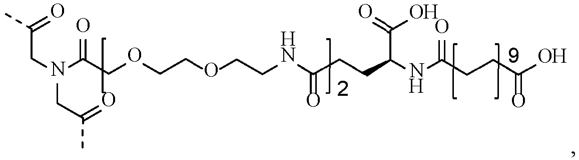 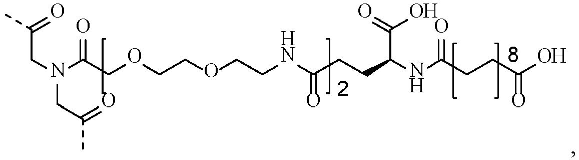 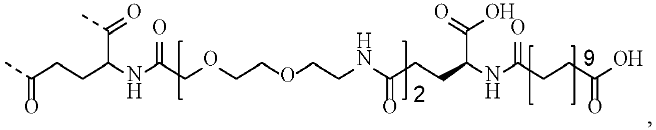 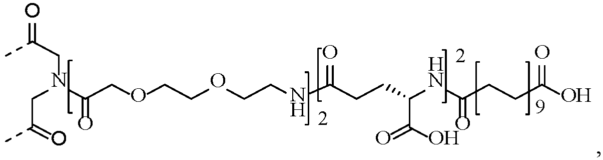 and

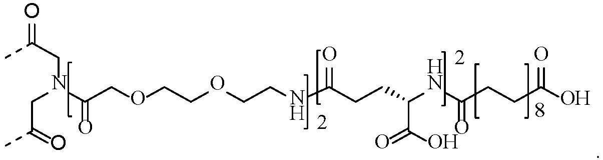

16. A pharmaceutical use of a polypeptide having a sequence selected from the group consisting of sequences as shown below in the preparation of a drug for treating and/or preventing diabetes, obesity, and related diseases thereof, 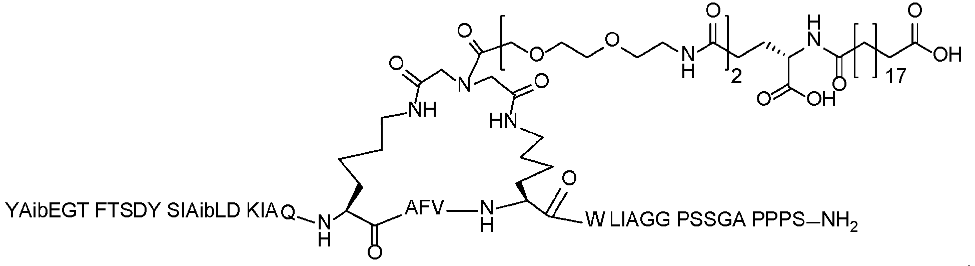 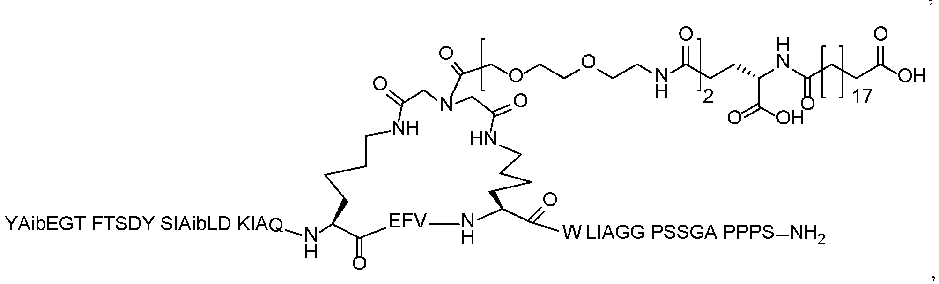 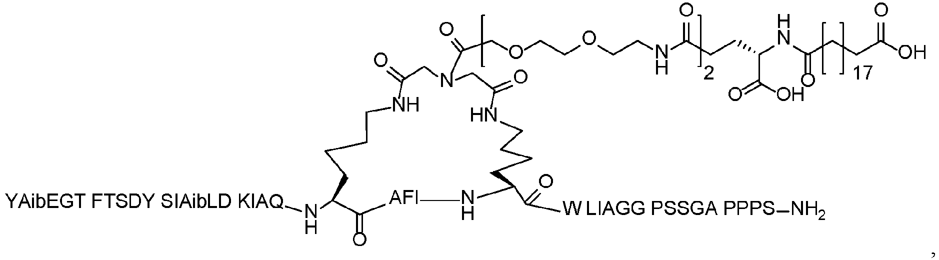 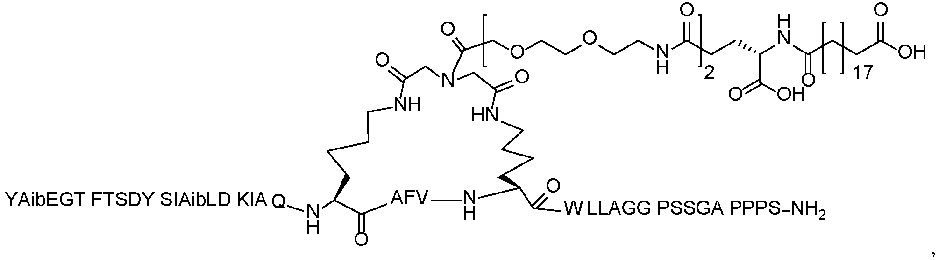 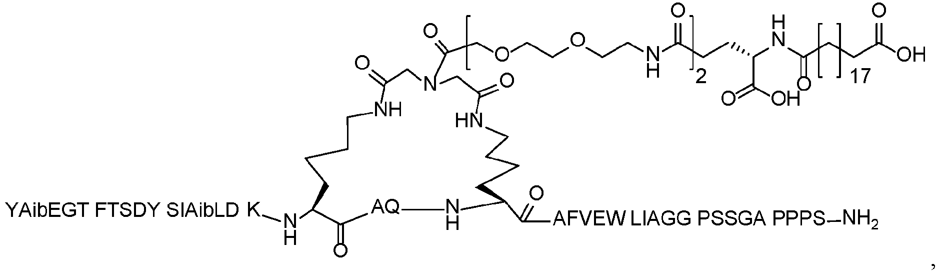 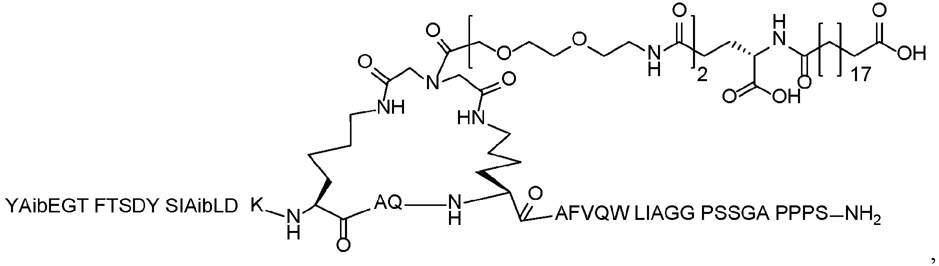 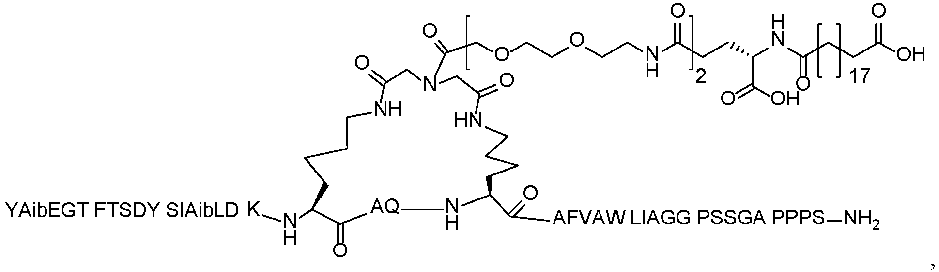 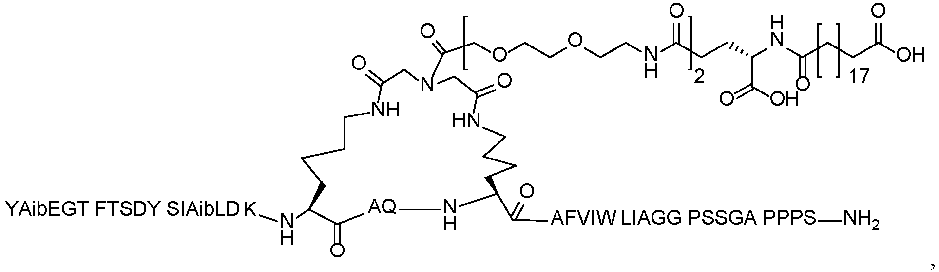 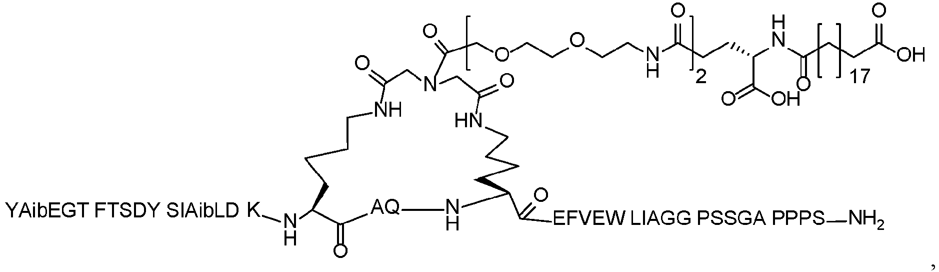 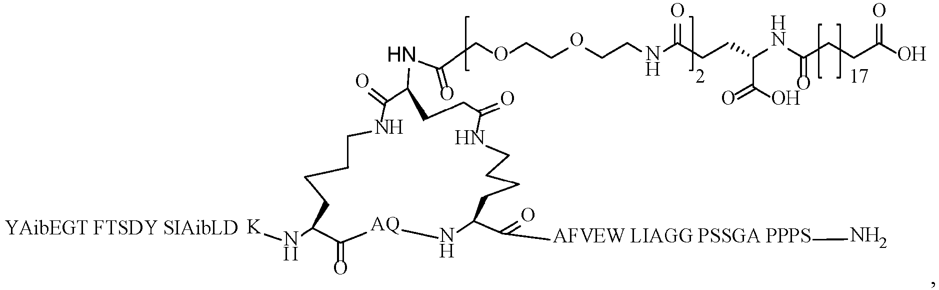 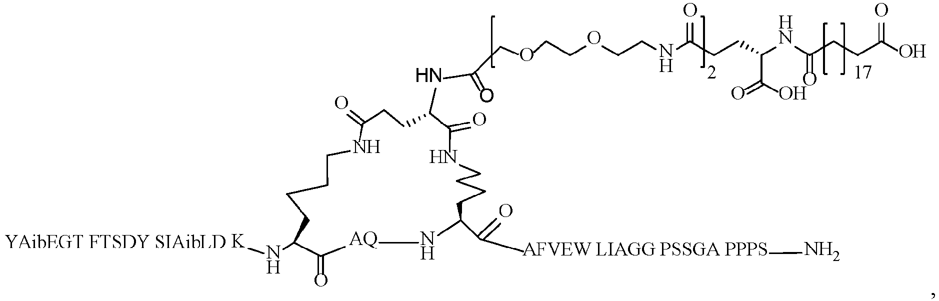 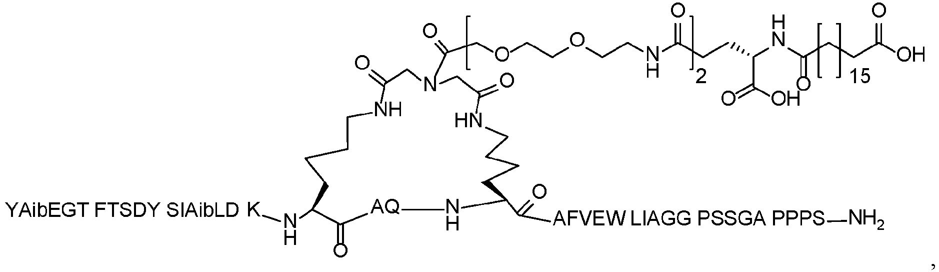 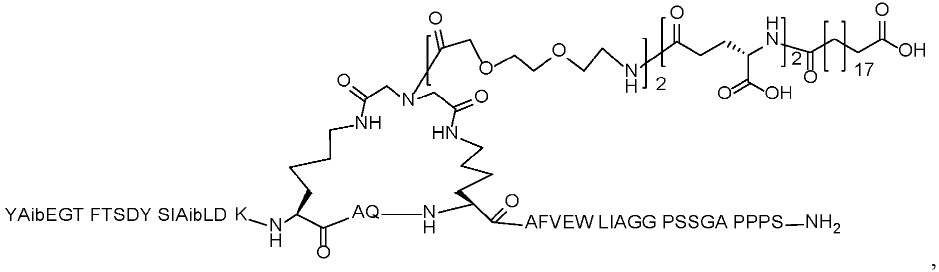 and

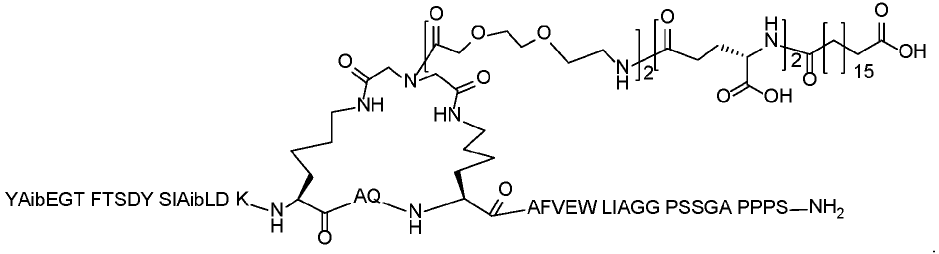

17. A pharmaceutical composition for treating and/or preventing diabetes, obesity, and related diseases thereof, wherein the pharmaceutical composition comprises a therapeutically effective amount of the compound of any one of claims 1 to 16 or a pharmaceutically acceptable salt thereof, as an active ingredient, and a pharmaceutically acceptable carrier.

18. The pharmaceutical use of the polypeptide or the pharmaceutically acceptable salt thereof in the preparation of a drug for treating and/or preventing diabetes, obesity, and related diseases thereof according to any one of claims 1 to 16, or the pharmaceutical composition for treating and/or preventing diabetes, obesity, and related diseases thereof according to claim 17, wherein the related diseases of diabetes comprise diabetic cardiovascular diseases, diabetic cerebrovascular diseases, retinopathy, nephropathy, cataracts, coronary artery diseases, diabetic neuropathy, diabetic foot, and peripheral neuropathy, and the related diseases of obesity comprise liver diseases, hyperlipidemia, hypertension, and other related diseases.

19. The pharmaceutical use of the polypeptide or the pharmaceutically acceptable salt thereof in the preparation of a drug for treating and/or preventing diabetes, obesity, and related diseases thereof according to any one of claims 1 to 16, or the pharmaceutical composition for treating and/or preventing diabetes, obesity, and related diseases thereof according to claim 17, wherein the drug for treating and/or preventing diabetes, obesity, and related diseases thereof is administered once every 3 days, once every 4 days, once a week, or once every two weeks.
